(19) Europäisches Patentamt
European Patent Office
Office européen des brevets

(11) EP 1 693 451 B1

(12) EUROPEAN PATENT SPECIFICATION

(45) Date of publication and mention of the grant of the patent:
**09.11.2011 Bulletin 2011/45**

(21) Application number: **04818986.4**

(22) Date of filing: **19.11.2004**

(51) Int Cl.:
*C12N 15/09* (2006.01) *C12N 5/10* (2006.01)
*A61K 31/7088* (2006.01) *A61K 35/34* (2006.01)
*A61K 35/76* (2006.01) *A61K 48/00* (2006.01)
*A61P 9/00* (2006.01) *A61P 9/04* (2006.01)
*A61P 9/10* (2006.01)

(86) International application number:
**PCT/JP2004/017274**

(87) International publication number:
**WO 2005/049822 (02.06.2005 Gazette 2005/22)**

# (54) METHOD OF GROWING MYOCARDIAL CELLS

VERFAHREN ZUR ANZUCHT VON MYOKARDZELLEN

PROCEDE DE CULTURE DE CELLULES MYOCARDIQUES

(84) Designated Contracting States:
**AT BE BG CH CY CZ DE DK EE ES FI FR GB GR HU IE IS IT LI LU MC NL PL PT RO SE SI SK TR**

(30) Priority: **21.11.2003 JP 2003391708**
**26.08.2004 JP 2004246533**

(43) Date of publication of application:
**23.08.2006 Bulletin 2006/34**

(73) Proprietor: **Daiichi Sankyo Company, Limited**
**Chuo-ku**
**Tokyo 103-8426 (JP)**

(72) Inventors:
- **ADACHI, Mimi**
  **Nakano-ku, Tokyo 164-0003 (JP)**
- **NAKAYAMA, Keiichi**
  **Fukuoka-shi,**
  **Fukuoka 812-0053 (JP)**
- **KITAJIMA, Shigetaka**
  **Kawasaki-shi,**
  **Kanagawa 216-0033 (JP)**
- **TAKAGI, Hiromitsu**
  **Takatsuki-shi, Osaka 5691118 (JP)**

(74) Representative: **Vossius & Partner**
**Siebertstrasse 4**
**81675 München (DE)**

(56) References cited:
- **LI J -M ET AL: "Cell cycle regulatory molecules (cyclins, cyclin-dependent kinases and cyclin-dependent kinase inhibitors) and the cardiovascular system: Potential targets for therapy?" EUROPEAN HEART JOURNAL, vol. 20, no. 6, March 1999 (1999-03), pages 406-420, XP002453201 ISSN: 0195-668X**
- **FLINK IRWIN L ET AL: "Changes in E2F complexes containing retinoblastoma protein family members and increased cyclin-dependent kinase inhibitor activities during terminal differentiation of cardiomyocytes" JOURNAL OF MOLECULAR AND CELLULAR CARDIOLOGY, vol. 30, no. 3, March 1998 (1998-03), pages 563-578, XP002453202 ISSN: 0022-2828**
- **TAMAMORI-ADACHI MIMI ET AL: "Down-regulation of p27Kip1 promotes cell proliferation of rat neonatal cardiomyocytes induced by nuclear expression of cyclin d1 and CDK4 - Evidence for impaired Skp2-dependent degradation of p27 in terminal differentiation" JOURNAL OF BIOLOGICAL CHEMISTRY, vol. 279, no. 48, 26 November 2004 (2004-11-26), pages 50429-50436, XP002453203 ISSN: 0021-9258**
- **TAMAMORI-ADACHI M. ET AL. DAI 26 KAI THE MOLECULAR BIOLOGY SOCIETY OF JAPAN NENKAI PROGRAM.KOEN YOSHISHU 25 November 2003, page 438, XP002988171**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 693 451 B1

- TAMAMORI-ADACHI M. ET AL.: 'Critical role of cyclin D1 nuclear import in cardiomyocyte proliferation' CIRC. RES. vol. 92, no. 1, January 2003, pages E12 - 19, XP002986988
- POOLMAN R.A. ET AL.: 'Altered expression of cell cycle proteins and prolonged duration of cardiac myocyte hyperplasia in p27KIP1 knockout mice' CIRC. RES. vol. 85, no. 2, 1999, pages 117 - 127, XP002986989
- KIM M.H. ET AL.: 'Successful inactivation of endogenous Oct-3/4 and c-mos genes in mouse preimplantation embryos and oocytes using short interfering RNAs' BIOCHEMICAL AND BIOPHYSICAL RESEARCH COMMUNICATIONS vol. 296, no. 5, 2002, pages 1372 - 1377, XP002986990
- TOYOSHIMA H. ET AL.: 'p27, a novel inhibitor of G1 cyclin-Cdk protein kinase activity, is related to p21' CELL vol. 78, no. 1, 1994, pages 67 - 74, XP001008808
- TSVETKOV L.M. ET AL.: 'p27(Kip1) ubiquitination and degradation is regulated by the SCF(Skp2) complex through phosphorylated Thr187 in p27' CURR. BIOL. vol. 9, no. 12, 1999, pages 661 - 664, XP000953301

## Description

The present invention relates to a method for proliferating mammalian cardiomyocytes.

Because adult cardiomyocytes lose their capacity to proliferate by cell division, the heart damage caused by exposure to various stresses such as ischemia and inflammation, leads to necrosis or loss of cardiomyocytes without compensation thereof. Consequently, survived cardiomyocytes are hypertrophed in a compensatory fashion to retain the cardiac functions "The review article Li and Brooks (1999), European Heart Journal 20:406-420 provides an overview of the cell cycle machinery and discusses how this controls the growth of cardiomyocytes." When the state is sustained over the permissible range of cardiomyocytes, however, the cardiomyocytes are further exhausted and killed. Finally, such state causes the deterioration of cardiac muscle functions, namely heart failure.

Heart diseases mainly including heart failure occupy the second position in the causes of the mortalities in Japan. Additionally, the prognosis of patients with heart diseases is so extremely poor that the 5-year survival is just at about 50 %. Therefore, it is believed that the development of an efficacious therapeutic method of heart failure is greatly advantageous from the standpoints of medical welfare and medical economy. Conventional therapeutic drugs for heart failure include digitalis preparations that increase the contractive force of the myocardium and xanthine preparations and other heart stimulants, but long-term administration of these drugs is known to make the condition worse. In recent years, therapeutics with pharmaceutical 1 agents to reduce excess cardiac burdens due to the elevation of the sympathetic nerve system and the renin-angiotensin system, such as β blockers and ACE inhibitors are in the mainstream. However, these therapeutic methods are simple symptomatic treatments and can never recover damaged cardiac tissues themselves. Alternatively, cardiac transplantation is an essential therapeutic method for severe heart failure. Due to problems such as the shortages of organ donors, medical ethics, and body burdens and economical burdens to patients, further, it is very tough to employ cardiac transplantation as a general therapeutic method.

Recently, a method of supplemental engraftment of cardiomyocytes to the injured heart has been studied. It is known that at some experiments using animals where cardiomyocytes obtained from fetuses were engrafted into an adult cardiac tissue, the engrafted cardiomyocytes could effectively function as cardiomyocytes (for example, see non-patent reference 1). Research works have been under way, so as to prepare cardiomyocytes from multi-potent cells with a potency to be differentiated into a wide variety of cells including cardiomyocytes, namely so-called embryonic stem cells (ES cells) for use as engrafting cells. However, these methods would face much difficulties in the application thereof to clinical medicine from an ethical standpoint. Recently, further attempts have been made to engraft bone marrow-derived stem cells to allow the stem cells to be differentiated into cardiomyocytes in cardiac tissue. However, the differentiation efficiency is extremely low. Therefore, the method is not practical as a method for the regeneration and compensation of cardiomyocytes (see for example non-patent references 2, 3 and 4 as reviews).

The present inventors of the invention therefore made research works about the cell cycle regulatory mechanism of cardiomyocytes, particularly the role of cyclin-cyclin-dependent kinase (CDK) system. Consequently, the inventors found that although the expression of the type D cyclin and CDK4 is induced via the stimulation of, for example, serum and growth factors in cardiomyocytes, these protein molecules are localized in cytoplasm but not transfered into nucleus, so that the phosphorylation of RB protein as a nuclear target molecule of cyclin D-CDK4 or the activation of cyclin E-CDK2 hardly occurs. When the inventors prepared an adenovirus vector where a gene for cyclin D1 tagged with the nuclear localization signal (NLC) (referred to as D1NLS hereinafter) and the gene encoding CDK4 were integrated to allow the vector to infect cultured cardiomyocytes, the cyclin D1 protein and the CDK4 protein were expressed in the nucleus, to cause the proliferation and division of cardiomyocytes via RB phosphorylation. Consequently, the inventors successfully allowed the proliferation of cardiomyocytes that hardly proliferate (mitotically divide) undergeneral culture conditions. Additionally, the inventors introduced D1NLS and the CDK4 gene in the cardiac muscle tissue of an adult animal to allow the expression thereof, so that the inventors successfully progressed the cell cycle of the cardiomyocytes of the adult animal (see for example patent reference 1, non-patent reference 5). The method for proliferating cardiomyocytes in accordance with the invention is now referred to as DNLS/CDK method hereinbelow.

A great number of other attempts were made to progress the cell cycle of cardiomyocytes to induce the division of the cells (see for example non-patent reference 6 as a review about the progress of the cell cycle of cardiomyocytes). For example, a report tells that when adenovirus-derived E1A/E1B gene (see non-patent reference 7) or E2F gene (see non-patent reference 8) was expressed in cultured cardiomyocytes isolated from a neonatal rat, the induction of DNA synthesis in the cardiomyocytes occurred. In a transgenic mouse excessively expressing the cyclin D1 gene of the intact type without any nuclear localization signal, the elevation of the expression level of CDK4 in the cardiomyocytes is observed together with the induction of DNA synthesis (see for example non-patent reference 9). More recently, it is indicated that in a mouse deficient in the jumonji gene suppressing the expression of the cyclin D1 gene, it is observed that the duration of the cell proliferation arrest in the fetal cardiomyocytes is prolonged (see for example non-patent reference 10). As described above, it is demonstrated that the induction of DNA synthesis and cell cycle progression may be possible even in cardiomyocytes. In the experimental examples, however, apoptosis induction and the appearance of abnormal polynuclear cells were frequently observed in cardiomyocytes. Except the DNLS/CDK method, no method exists for promoting cell division of cardiomyocytes after birth to practically increase cell number.

As described above, the DNLS/CDK method is an innovative method by which cardiomyocytes generally "hardly proliferating" can be increased. Thus, the method is industrially highly applicable.

It is known that plural up- and down-regulatory factors regulate the progress of cell cycles in general eukaryotic cells. The up-regulatory factors include the individual types of cyclin and CDK, while the down-regulatory factors include a series of protein groups called CDK-inhibitors. Two families of CDK-inhibitors are identified, which have different action modes from each other (see for example non-patent reference 11 as a review). A first group is called Ink4 family protein and includes p16 (also known as Ink4A, Mts1, Cdkn2 and Cdkn4i), p15 (also known as Ink4B and Mts2), p18 (also known as Ink4C and Ink6A), and p19 (also known as p20, Ink4D and Ink6B). The first group selectively binds to CDK4 or CDK6 to inhibit the function of the cyclin D-CDK4 (or CDK6) complex (see for example non-patent references 12, 13 and 14). A second group of CDK-inhibiting proteins is called Cip/Kip family protein and includes p21 (also known as Cip1, Pic1, Sdi1, mda6 and Waf1; referred to as $p21^{Cip1}$ hereinafter), p27 (also known as Ick, Kip1 and Pic2; referred to as $p27^{Kip1}$ hereinafter) , and p57 (also known as Kip2; referred to as $p57^{Kip2}$ hereinafter). It is shown that unlike the Ink4 family, the second family inhibits the progress of cell cycle by inhibiting the functions of various cyclin-CDK complexes (see for example non-patent references 15, 16, 17 and 18).

As to how these CDK-inhibiting proteins are involved in the suppression of the proliferation of cardiomyocytes, several reports tell about the Cip/Kip family molecules. Specifically, it is known that the expression levels of the $p21^{Cip1}$ protein and the $p27^{Kip1}$ protein are elevated, following the deterioration of the proliferation potency of cardiomyocytes over the late fetal stage to postnatal stage, so that the activation of CDK2 and CDK4 as target molecules is lowered (see for example non-patent reference 19). When IGF-1 (insulin-like growth factor-1) is added to cardiomyocytes with excess expres-sion of the E2F-1 gene therein, the expression levels of the $p21^{Cip1}$ protein and the $p27^{Kip1}$ protein are lowered, involving the increase of the ratio of cardiomyocytes at the DNA synthetic phase (the S phase) (see for example non-patent reference 20). It is also reported that in a $p27^{Kip1}$ gene-deficient mouse, the timing of the proliferation arrest of cardiomyocytes is delayed than in normal mouse and that the number of cardiomyocytes in the deficient mouse is increased (see for example non-patent reference 21). As described above, it is suggested that the Cip/Kip family protein, particularly the $p27^{Kip1}$ protein may possibly be involved in the suppression of the proliferation of cardiomyocytes. No example except for deletion of the gene is known for suppressing the expression and function of the Cip/Kip family protein to induce the division and proliferation of cardiomyocytes.

It is known that the intracellular expression level of the Cip/Kip family protein is mainly regulated by a degradation system via the ubiquitin pathway (see for example non-patent references 22, 23 and 24). Ubiquitin is a polypeptide comprising 76 amino acids highly preserved and is abundant in all eukaryotic cells. In the ubiquitin pathway, polyubiquitin chain covalently binds to a target substrate and is subsequently degraded with a polyfunctional proteasome complex. Protein molecules degraded with such ubiquitin-proteasome system include a wide variety of molecules such as cyclin, p53, p300, E2F, STAT-1, c-Myc, c-Jun, EGF receptor, IκBα, NFκB and β-catenin, in addition to the Cip/Kip family protein. Intensive research works on the ubiquitinylation mechanism of protein molecules are now under way. Generally, protein molecules are ubiquitinylated by a series of enzyme groups, namely ubiquitin activation enzyme (E1), ubiquitin complexing enzyme (E2) and ubiquitin ligase (E3), and ubiquitinylated proteins are finally degraded with 26S proteasome (see for example non-patent references 25, 26, 27, and 28 as reviews).

It is believed that, the ubiquitin ligase (E3) is responsible for the specificity of the ubiquitinylation of specific target proteins among them. Numerous examples are known, such as Anaphase Promoting Complex/Cyclosome (APC/C) complex, VHL (von Hipple-Lindau protein-elongin B/C (VBC) complex, Nedd4, Ufd4, Rad5, Rad18 and Parkin. A new type of ubiquitin ligase complex called SCF has been identified recently through research works on lower biological organisms such as yeast. The ubiquitin ligase of the SCF complex type (sometimes referred to as ubiquitin ligase SCF complex hereinafter) is a protein module of a trimer composed of three subunits called Skp1, Cull (a different name of Cdc53), and F-box protein. The ligase is called SCF as an acronym of the individual subunits (see for example non-patent references 29 and 30 as reviews)).

The F-box protein as one of the components of SCF complex contains an F-box motif first identified in cyclin F. The motif region is required for the interaction with Skp1. Additionally, the F-box protein contains a repeat motif region of about 40 amino acid sequences as called WD-40 repeat or a leucine-enriched motif region called leucine-rich repeat. In the SCF complex, Skp1 and Cull/Cdc53 are never variable against any target substrates, while the molecular species of the F-box protein vary, depending on the target substrate as an ubiquitinylation target. By recognizing and binding a target substrate in the WD-40 repeat or the leucine-rich repeat, the F-box protein determines the substrate specificity of the SCF complex (see for example non-patent references 31, 32, and 33). As described above, the SCF complex includes plural types of $SCF^{\beta TrCP}$, $SCF^{Cdc4}$, $SCF^{Met30}$ and $SCF^{Grr1}$, depending on the difference of the F-box protein contained as the component (see for example non-patent references 29 and 30).

In case of the Cip/Kip family protein, an SCF complex containing Skp2 ($SCF^{Skp2}$) as an F-box protein is involved in the ubiquitin-proteasome degradation thereof. Skp2 was first identified as a factor binding to the cyclin A-CDK2 complex. Because the accumulation of Skp2 occurs from the late G1 phase of cell cycle and the expression level reaches the maximum from the S phase to the G2 phase, Skp2 is called S-phase kinase-associated protein (see for example non-patent reference 38). A report tells that other than the Cip/Kip family protein, Skp2 recognizes protein molecules such as E2F-1 (see non-patent reference 39), cyclin E (see non-patent reference 40), CDK9 (see non-patent reference 41) and c-Myc (see non-patent references 42 and 43) as target substrates and Skp2 is involved in the degradation thereof.

As described above, it is known that CDK-inhibitors including the Cip/Kip family protein are involved in the suppression of the proliferation of general proliferative cells, so that the ubiquitin-proteasome system via the $SCF^{Skp2}$ complex is responsible for the regulation of the intracellular expression level. However, almost nothing is known about how the ubiquitin-proteasome system is involved in the regulation mechanism of the proliferation of cardiomyocytes.

The DNLS/CDK method is a method for proliferating cardiomyocytes as a currently known single one such method. The method is very useful and highly industrially applicable. So as to promote the practical application of cardiac muscle regeneration therapy and the industrial applicability thereof, still further, the effect of proliferating cardiomyocytes and the efficiency thereof are desirably raised.

Patent reference 1: Pamphlet of International Publication WO 02/095026
Non-patent reference 1: Soonpaa, et al., Science 264:98, (1994)
Non-patent reference 2: Murry, et al., Cold Spring Harb. Symp. Quant. Biol. 67:519, (2002)
Non-patent reference 3: Menasche, Ann. Thorac. Surg. 75:S20, (2003)
Non-patent reference 4: Nir, et al., Cardiovasc. Res. 58:313, (2003)
Non-patent reference 5: Tamamori-Adachi, et al., Circ. Res. 92:e12, (2003)
Non-patent reference 6: Kishore, et al., Circ. Res. 90:1044, (2002)
Non-patent reference 7: Kirshenbaum, et al., J. Biol. Chem. 270: 7791, (1995)
Non-patent reference 8 : Kirshenbaum, et al., Dev. Biol. 179:402, (1996)
Non-patent reference 9: Soonpaa, et al., Clin. Invest. 99: 2644, (1997)
Non-patent reference 10: Toyoda, et al., Dev. Cell 5: 85, (2003)
Non-patent reference 11: Sherr & Roberts, Genes Dev. 9:1149, (1995)
Non-patent reference 12: Hannon& Beach, Nature 371: 257, (1993)
Non-patent reference 13: Serrano, et al., Nature 366: 704, (1993)
Non-patent reference 14: Hirai, et al., Mol. Cell. Biol. 15:2672, (1995)
Non-patent reference 15: Harper, et al., Cell 75:805, (1993)
Non-patent reference 16: Polyyak, et al., Cell 78:59, (1994)
Non-patent reference 17: Toyoshima & Hunter Cell 78:67, (1994)
Non-patent reference 18: Matsuoka, et al., Genes Dev. 9:650, (1995)
Non-patent reference 19: Flink, et al., J. Mol. Cell. Cardiol. 30:563, (1998)
Non-patent reference 20: von Harsdorf, et al., Circ. Res. 85:128, (1999)
Non-patent reference 21: Poolman, et al., Circ. Res. 85:117, (1999)
Non-patent reference 22: Pagano, et al., Science 269: 682 (1995)
Non-patent reference 23: Maki& Howley, Mol. Cell Biol. 17:355, (1997)
Non-patent reference 24: Urano, et al., J. Biol. Chem. 274:12197, (1999)
Non-patent reference 25: Coux, et al., Annu. Rev. Biochem. 65:801, (1996)
Non-patent reference 26: Hochstrasser, Annu. Rev. Genet. 30:405, (1996)
Non-patent reference 27: Pagano, FASEV J. 11:1067, (1997)
Non-patent reference 28: Hershko, et al., Annu. Rev. Biochem. 67:425, (1998)
Non-patent reference 29: Patton, et al., Trends Genet. 14:236, (1998)
Non-patent reference 30: Jackson & Eldridge, Mol. Cell 9:923,(2002)
Non-patent reference 31 : Bai, et al., Cell 86:263, (1996)
Non-patent reference 32: Slowrya, et al., Cell 91:209, (1997)
Non-patent reference 33: Kobe, et al., Curr. Opin. Struct. Biol. 11725, (2001)
Non-patent reference 34: Carrano, et al., Nature Cell Biol. 1:193, (1999)
Non-patent reference 35: Tsverkov, et al., Curr. Biol. 9:661, (1999)
Non-patent reference 36: Bornstein, et al., J. Biol . Chem. 278:26752, (2003)
Non-patent reference 37: Kamura, et al., Proc. Natl . Acad. Sci. USA 100:10231, (2003)
Non-patent reference 38: Zhang, et al., Cell 82:915, (1995)
Non-patent reference 39: Marti, et al., Nat. Cell Biol. 1:14, (1999)
Non-patent reference 40 : Nakayama, et al., EMBO J. 19:2069, (2000)
Non-patent reference 41: Kiernan, et al., Mol. Cell. Biol. 21:7956, (2001)
Non-patent reference 42: Kim, et al., Mol. Cell 11:1177, (2003)
Non-patent reference 43: von der Lehr, et al., Mol. Cell 11:1189, (2003)

In a method for proliferating cardiomyocytes, it is an object of the present invention to provide a method for enhancing the proliferation efficiency of the cardiomyocytes, and to provide a recombinant vector and the like for use in the method.

So as to solve the problems, the inventors analyzed the mechanism of the cell cycle regulation in cardiomyocytes. Specifically, the inventors examined the mechanism therein with attention focused on the performance of individual factors for the cell cycle regulation in cardiomyocytes with enforced expression of cyclin and CDK genes, particularly CDK-inhibitors therein. Consequently, the inventors found that one of the Cip/Kip family protein as the CDK-inhibitor, namely $p27^{Kip1}$ was unexpectedly accumulated excessively in the nucleus of cardiomyocytes under stimulation with cyclin and CDK.

It is known that the Cip/Kip family protein mainly including $p27^{Kip1}$ is ubiquitinylated with ubiquitin ligase in general proliferating cells, so that the Cip/Kip family protein is degraded with proteasome. Therefore, the gene encoding a component of ubiquitin ligase was co-expressed with the cyclin and CDK genes in cardiomyocytes. It was then observed that the $p27^{Kip1}$ protein was significantly reduced in the nucleus of cardiomyocytes. In addition, the cell proliferation potency of cardiomyocytes was found to be highly activated, and these findings have led to the completion of the present invention.

Specifically, the present invention relates to a method as defined by the claims to increase the proliferation efficiency of cardiomyocytes by inhibiting the production, function and action (effect) of the Cip/Kip family protein expressed in cardiomyocytes on stimulation with cyclin and CDK. The Cip/Kip family protein to be suppressed of the action is not limited to but, the protein is preferably $p27^{Kip1}$.

In accordance with the invention, the term "cardiomyocytes" means any cardiac muscle cells expressing plural markers specific to cardiomyocytes, which are recognized as the morphological, physiological and/or immunological features of intact cardiomyocytes. The term includes not only cardiomyocytes directly obtained from cardiac tissues of mammals and primary cultured cells thereof but also cardiomyocytes differentiated and derived from stem cells such as embryonic stem cells, bone marrow mesenchymal stem cells and CMG cells.

Any method for inhibiting the production, function and action of the Cip/Kip family protein may be satisfactory with no specific limitation. The method works for suppressing the expression of the gene encoding the protein, suppressing the production of the protein, inhibiting the activity of the protein or promoting the degradation of the protein.

In particular, the method for promoting the degradation of the protein is preferably a method for promoting the ubiquitinylation of the protein. The ubiquitinylation can be done by introducing for example drugs, proteins, peptides, low molecular compounds, and genes into a target cell.

Additionally, the gene promoting the ubiquitinylation of the Cip/Kip family protein is preferably _ a gene encoding a component of ubiquitin ligase, more preferably a gene encoding the F-box factor capable of binding to the Cip/Kip family protein, including for example Skp2 gene.

In carrying out the invention, furthermore, a method for inhibiting the expression (mRNA transcription) of the gene encoding the Cip/Kip family protein or inhibiting the translation and production of the gene product may also be used. For example, siRNA specific to the gene encoding the Cip/Kip family protein is preferably used.

In carrying out the invention, additionally, a nucleotide sequence encoding the nuclear localization signal is preferably tagged to at least one of the cyclin gene and the CDK gene, to introduce the resulting gene into the target cells. In accordance with the invention, the cyclin is a cyclin capable of activating CDK4 or CDK6 namely cyclin D1, D2 and D3 of mammals. Also in accordance with the invention, the CDK is activated by type D cyclin, namely CDK4 and CDK6 of mammals.

In another embodiment, the invention relates to a vector as defined by the claims carrying the cyclin gene, the CDK gene and the gene of a factor inhibiting the action of the Cip/Kip family protein. In case of intending the introduction of the gene into cardiomyocytes, preferably, the introduction is done, using a viral vector or liposome or the like. As the viral vector, for example, adenovirus vector is preferably used.

In an additional mode for carrying out the invention, the invention relates to a pharmaceutical composition containing a vector carrying the cyclin gene, the CDK gene and the gene of a factor inhibiting the action of the Cip/Kip family protein.

In a still additional mode for carrying out the invention, the invention relates to cardiomyocytes obtained by the method for proliferating cardiomyocytes.

Also described herein is a screening method using a cell obtained by the method for proliferating cardiomyocytes, so as to identify a novel factor maintaining and promoting the viability and function of cardiomyocytes and the like or a chemotherapeutic agent with a possibility thereof.

In an additional mode for carrying out the invention, the invention relates to a therapeutic use of treating heart diseases, wherein the pharmaceutical composition as defined by the claims is to be administered into a site in a patient where the cardiomyocytes are weakened, functionally arrested or killed, to retain and proliferate the cells.

Accordingly, the present invention relates to the following matters.

(1) A pharmaceutical composition comprising:

(a) a cyclin, said cyclin being selected from cyclin D1, cyclin D2, and cyclin D3,
(b) a cyclin-dependent kinase, said cyclin-dependent kinase being selected from CDK4 and CDK6, and
(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family protein;

wherein at least one of the gene encoding said cyclin and the gene encoding said cyclin-dependent kinase is tagged with a nucleotide sequence encoding a nuclear localization signal.

(2) A method for proliferating cardiomyocytes comprising a step of introducing

(a) a cyclin, said cyclin being selected from cyclin D1, cyclin D2, and cyclin D3,
(b) a cyclin-dependent kinase, said cyclin-dependent kinase being selected from CDK4 and CDK6, and
(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family protein

into cardiomyocytes in vitro; and
a step of subsequently culturing said cells;
wherein at least one of the gene encoding said cyclin and the gene encoding
said cyclin-dependent kinase is tagged with a nucleotide sequence encoding a nuclear localization signal.

(3) Use of

(a) a cyclin, said cyclin being selected from cyclin D1, cyclin D2, and cyclin D3,
(b) a cyclin-dependent kinase, said cyclin-dependent kinase being selected from CDK4 and CDK6, and
(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family protein

for the manufacture of a pharmaceutical composition for treating a cardiac disorder,
wherein at least one of the gene encoding said cyclin and the gene encoding said cyclin-dependent kinase is tagged with a nucleotide sequence encoding a nuclear localization signal.

(4) The pharmaceutical composition of (1), the method of (2), or the use of (3), wherein the Cip/Kip family protein is $p27^{Kip1}$.

(5) The method of (2), comprising introducing the genes into cardiomyocytes, using a viral vector or liposome.

(6) A vector comprising

(a) a cyclin gene, the cyclin being selected from cyclin D1, cyclin D2, and cyclin D3,
(b) a cyclin-dependent kinase gene, the cyclin-dependent kinase being selected from CDK4 and CDK6, and
(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and

(ii) a nucleic acid that inhibits the production of Cip/Kip family protein, wherein said nucleic acid is siRNA specific to a gene encoding the Cip/Kip family protein,

wherein at least one of the gene encoding said cyclin and the gene encoding said cyclin-dependent kinase is tagged with a nucleotide sequence encoding a nuclear localization signal.

(7) The pharmaceutical composition of (1) or (4), the method of any one of (2), (4) or (5), the use of (3) or (4), or the vector of (6), wherein the component of ubiquitin ligase is an F-box factor capable of binding to the Cip/Kip family protein.

(8) The pharmaceutical composition, method, use or vector of (7), wherein the F-box factor capable of binding to the Cip/Kip family protein is Skp2.

(9) The pharmaceutical composition of (1) or (4), the method of any one of (2), (4) or (5), the use of (3) or (4), or the vector of any one of (6) to (8), wherein the nucleic acid that inhibits the production of Cip/Kip family protein is siRNA that is specific to $p27^{Kip1}$ gene.

(10) A pharmaceutical composition comprising the vector of any one of (6) to (9).

(11) Use of the vector of any one of (6 to (9 for the manufacture of a pharmaceutical composition for treating a cardiac disorder.

(12) The use of any one of (3), (4), (7) to (9) or (11), wherein the cardiac disorder is myocardial infarction, ischemic heart disease, congestive heart failure, hypertrophic cardiomyopathy, dilated cardiomyopathy, myocarditis, or chronic heart failure.

The use of the method in accordance with the invention enables the inhibition of the action of the Cip/Kip family protein in the nucleus of cardiomyocytes, to promote the proliferation of cardiomyocytes. Further, the advantages and characteristic features of the invention are sufficiently described below in the Section Detailed Description of Preferable Mode for Carrying out the Invention.

## BRIEF DESCRIPTION OF DRAWINGS

[Fig. 1]
Expression of $p27^{Kip1}$ protein in cardiomyocytes transfected with D1NLS and CDK4 genes. Preparing the nuclear protein, the expression of $p27^{Kip1}$ protein was examined by Western blotting. CM: rat cardiomyocytes; REF: rat fibroblast cell line (REF52 cell).
[Fig.2]
Localization of $p27^{Kip1}$ protein in cardiomyocytes transfected with D1NLS and CDK4 genes. Infecting cardiomyocytes with a recombinant adenovirus carrying the LacZ gene (upper column; Cont) or D1NLS gene and CDK gene (lower column), the intracellular expression of $p27^{Kip1}$ protein was examined by immuno-fluorescent staining method. In the figure, the green color expresses $p27^{Kip1}$ and the red color expresses sarcomeric actin. Further, the cell nuclei were stained with DAPI.
[Fig.3]
Regulation of the expression of $p27^{Kip1}$ protein in cardiomyocytes. Cardiomyocytes under stimulation with FBS or stimulation with D1NLS + CDK4 was treated with a proteasome inhibitor lactastatin (LC) for comparison in terms of the expression of $p27^{Kip1}$ protein.
[Fig. 4]
Ubiquitinylation of $p27^{Kip1}$ protein in cardiomyocytes. A cell extract from cardiomyocyte (CM) or fibroblast cell (REF) under stimulation with FBS or stimulation with D1NLS + CDK4 was used for in vitro ubiquitinylation, to subsequently detect $p27^{Kip1}$ protein by Western blotting. In the figure, IB. expresses the antibody used for Western blotting analysis. A band observed at a position with a higher molecular weight is ubiquitinylated $p27^{Kip1}$ protein (p27-GST-Ub).
[Fig.5]

Expression of Skp2 protein in cardiomyocytes. Cardiomyocytes under stimulation with FBS or stimulation with D1NLS + CDK4 was treated with a proteasome inhibitor lactastatin (LC) for comparison in terms of the expression of Skp2 protein.

[Fig. 6]

Reduction of $p27^{Kip1}$ protein via the co-expression of the Skp2 gene. The expression of $p27^{Kip1}$ protein in cardiomyocytes transfected with D1NLS, CDK4 and Skp2 genes was examined.

[Fig. 7]

Localization of $p27^{Kip1}$ protein in cardiomyocytes with D1NLS, CDK4 and Skp2 genes co-expressed therein. Infecting cardiomyocytes with a recombinant adenovirus carrying D1NLS + CDK4 genes (upper column; Cont) or carrying D1NLS CDK + Skp2 genes (middle and lower columns) and treating a part of the cardiomyocytes with a proteasome inhibitor lactastatin (LC) (lower column), the intracellular expression of $p27^{Kip1}$ protein was examined by immunofluorescent staining method. In the figure, the green color expresses $p27^{Kip1}$ and the red color expresses sarcomeric actin. Further, the cell nuclei were stained with DAPI.

[Fig. 8]

Effect of enforced Skp2 gene expression on the promotion of the proliferation of cardiomyocytes. Infecting cardiomyocytes with a recombinant virus carrying LacZ gene (Cont), D1NLS + CDK4 genes, D1NLS + CDK4 + Skp2 genes or Skp2 gene alone, the number of cardiomyocytes was counted on the indicated days.

[Fig. 9]

Reduction of $p27^{Kip1}$ protein via the co-expression of siRNA specific to the $p27^{Kip1}$ gene. In cardiomyocytes transfected with D1NLS, CDK4 genes and siRNA specific to the $p27^{Kip1}$ gene, the expression of $p27^{Kip1}$ protein was examined.

[Fig. 10]

Effect of enforced expression of siRNA specific to the $p27^{Kip1}$ gene on the promotion of the proliferation of cardiomyocytes. Infecting cardiomyocytes with a recombinant virus carrying LacZ gene (Cont), D1NLS + CDK4 genes, D1NLS + CDK4 + siRNA specific to $p27^{Kip1}$ genes (p27 siRNA) or carrying siRNA specific to the $p27^{Kip1}$ gene alone, the number of cardiomyocytes was counted on the indicated days.

[Fig. 11]

Examination about the effect of enforced Skp2 gene expression on the reduction of lung weight. The lung weight of a rat 6 weeks post-cardiac ischemia and reperfusion was measured, to calculate the ratio to the body weight. : $p < 0.05$ vs. Sham group. $^{\#}$: $p < 0.05$ vs. Cont group.

[Fig.12]

The change of passive left ventricle-volume curve via the enforced expression of Skp2 gene. Using a rat heart 6 weeks after cardiac ischemia and reperfusion, a passive left ventricle-volume curve was prepared. : $p < 0.05$ vs. Sham group. $^{\#}$:$p < 0.05$ vs. Cont group. $^{\dagger}$ : $p < 0.05$ vs. D1NLS group.

[Fig. 13]

Examination about the effect of the enforced expression of Skp2 gene on the reduction of the area with cardiac infarction. The area with cardiac infarction in a rat heart 6 weeks after cardiac ischemia and reperfusion was measured to calculate the ratio occupying left ventricle. $^{\#}$: $p < 0.05$ vs. Cont group.

$^{+}$: Expressing addition of FBS and pharmaceutical agents or infection with various types of recombinant adenoviruses.

-: Expressing no addition of FBS and pharmaceutical agents or no infection with various types of recombinant adenoviruses.

p27: Expressing $p27^{Kip1}$ protein.

In carrying out the invention, persons intending the practice of the invention may see standard reference textbooks in the field of the art concerning molecular biology, microbiology, cell biology and general methods such as recombinant DNA technology and the related-art technology, unless otherwise stated. These include for example Molecular Cloning: A Laboratory Manual, the 3rd edition (Sambrook & Russel, Cold Spring Harbor Laboratory Press, 2001); Current Protocols in Molecular biology (edited by Ausubel, et al., John Wiley & Sons, 1987); Methods in Enzymology in series (Academic Press); PCR Protocols: Methods in Molecular Biology (edited by Barlett & Striling, Humana Press, 2003); Animal Cell Culture: A Practical Approach, the 3rd edition (edited by Masters, Oxford University Press, 2000); Antibodies: A Laboratory Manual (edited by Harlow, et al. & Lane, Cold Spring Harbor Laboratory Press, 1987). Reagents and kits for cell culture and cell biology experiments to be referred to in this specification may be commercially available from manufacturers such as Sigma, Aldrich, Invitrogen/GIBCO, Clontech, and Stratagene.

(Preparation of cardiomyocytes)

Cardiomyocytes as a target to be proliferated using the method of the invention includes all such cells at the stage of development, such as fetal type, neonatal type, and adult type cardiomyocytes and is defined as cells with at least one, preferably plural markers or standards, verified by at least one, preferably a plurality of the methods described below.

The expression of various markers specific to cardiomyocytes can be detected by conventional biochemical or immunochemical approaches. The approaches are not specifically limited. Preferably, immunochemical approaches such as immunocytochemical staining method and immunoelectrophoresis are used. For these approaches, marker-specific polyclonal antibodies or monoclonal antibodies reacted with cardiomyocyte precursor cells or cardiomyocytes may be used. Antibodies targeting individual specific markers are commercially available and may readily be used. Markers specific to cardiomyocyte precursor cells or cardiomyocytes include for example myosin heavy chain/light chain, $\alpha$-actinin, troponin I, ANP, GATA-4, Nkx2.5, and MEF-2c.

Otherwise, the expression of cardiomyocyte precursor cells- or cardiomyocyte-specific markers can be verified by molecular biology methods commonly used in the related art for amplifying, detecting and analyzing mRNA encoding an appropriate marker protein, including for example reverse transcriptase-mediated polymerase chain reaction (RT-PCR) and hybridization analysis. Nucleic acid sequences encoding marker proteins (for example, myosin heavy chain/light chain, $\alpha$-actinin, troponin I, ANP, GATA-4, Nkx2.5, and MEF-2c) specific to cardiomyocyte precursor cells or cardiomyocytes have been known and may be used from public databases such as GenBank at the National Center for Biotechnology information. Marker-specific sequences required for use as primers or probes can readily be determined.

Further, physiological indices may additionally be used. Specifically, the autonomous pulsation of cardiomyocytes and the expression of various ion channels in cardiomyocytes so that cardiomyocytes can react with electrophysiological stimuli are the useful indicators thereof.

According to the method disclosed in accordance with the invention, cardiomyocytes existing in heart tissues of mammals can be used directly as the subject. Additionally, cardiomyocytes separated from fresh heart tissues by various methods such as enzyme process or a primary culture cell thereof, which is obtained by culturing the cardiomyocytes under appropriate culture conditions for about one to 5 days may also be used. Specific culture methods of cardiomyocytes are described in numerous reference textbooks. Herein, typical methods thereof include the Chien's method and methods modified from the Chien's method (Chien, et al., J. Clin. Invest. 75: 1770, 1985; Meidell, et al., Am. J. Physiol. 251: H1076, 1986; Tamamori, et al., Am. J. Physiol. 275: H2036, 1998).

Additionally, the cultured cardiomyocytes includes cardiomyocytes obtained via the induction of the differentiation from stem cells, with no limitation to the example described above. The stem cells for use in carrying out the invention means cells with a property of possible differentiation into a cell with cardiomyocyte-like phenotypes under in vitro culturing. Specifically, the stem cells include for example embryonic stem cells (ES cells) and embryonic germ cells (EG cells) as derived from mammals such as mouse, monkey and human, for currently wide use as culture cells, and multi-potent stem cells such as adult type multi-potent adult progenitor cells (MAPC). Standard protocols for the methods for preparing, subculturing and storing these cells have been established. With reference to for example many reference textbooks such as Manipulating the Mouse Embryo: A laboratory manual (edited by Hogan, et al., Cold Spring Harbor Laboratory Press, 1994), Embryonic Stem Cells (edited by Turkesen, Humana Press, 2002) and plural textbooks (Matsui, et al., Cell 70:841, 1992; Shamblott, et al., Proc. Nat1. Acad. Sci. USA 95:13726, 1998; USP No. 6,090,622; Jiang, et al., Nature 418:41, 2002; International Publication No. 01/11011), these multi-potent stem cells can readily be used.

The stem cells usable in accordance with the invention is not limited to the three types described above. Any stem cells with features similar to those of the ES cells may be used. In this case, the features similar to those of ES cells can be defined as cell biological properties specific to ES cells, such as the existence of ES cell-specific surface (antigen) marker, the expression of ES cell-specific gene and teratoma-forming potency and chimera mouse-forming potency. Specific examples thereof include stem cells with features similar to those of ES cells, such as stem cells obtained by treating root sheath cell and epidermis cell with chemical agents such as 5-azacytidine (Sharda & Zahner, International Publication No. 02/051980), stem cells obtained by treating mononuclear cell with CR3/43 antibody (Abuljadayel, Curr. Med. Res. Opinion 19:355. 2003), and stem cells derived from adult inner ear cells (Li, et al., Nature Med., Advance online publication).

The method of the invention may be used for any cells without features similar to those of ES cells or any non-multi-potent cells, as long as the cell has at least a property to be differentiated into cells with cardiomyocyte-like phenotypes at least under culturing in vitro. Examples of such cells include mesenchymal stem cells derived from bone marrow cells (Bruder, et al., USP 5, 736, 396; Pittenger, et al., Science 284:143, 1999), CMG cells (Makino, et al., J. Clin. Invest. 103:697, 1999; International Publication WO 01/048151) and Spoc cells derived from muscle tissue (International Publication WO 03/035382).

As the culture method for preparing cardiomyocytes from stem cells in accordance with the invention, any method suitable for inducing the differentiation of cardiomyocytes may be used. In case of using for example ES cells, the culture method includes for example suspension culture method, hanging drop culture method, co-culture method with supporting cells, rotation culture method, soft agar culture method and micro-carrier culture method. Plural methods have been established for the specific method of inducing the differentiation. Persons intending the practice of the invention may see reference textbooks such as Embryonic Stem Cells (edited by Turksen, Human Press, 2002), and plural reference textbooks (Klug, et al., J. Clin. Invest. 98:216, 1996; Wobus, et al., J. Mol. Cell. Cardiol. 29:1525, 1997; Kehat, et al., J. Clin. Invest. 108:407, 2001;Xu, et al., Circ. Res. 91:501, 2002; Takahashi, et al., Circulation 107:1912, 2003; Field, et al., USP 6,015,671; Xu, et al., International Publication WO 03/06950).

(Method for proliferating cardiomyocytes)

One mode for carrying out the invention is a method as defined in the claims for proliferating cardiomyocytes including a step of introducing and expressing cyclin and CDK into cardiomyocytes and a step of inhibiting the production, function and action of the Cip/Kip protein. As the cardiomyocytes, cells separated from living cardiac tissues by various methods such as enzyme treatment, a primary culture cell obtained by culturing the aforementioned cardiomyocytes under appropriate culture conditions for about one to 5 days, and cardiomyocytes differentiated and induced from various stem cells may be used as described above. Cardiomyocytes existing in the cardiac tissue of a mammal can be proliferated by directly treating the cardiomyocytes by various methods described below and maintaining the cells in vivo. The term "maintaining" herein means allowing the cell to survive without any deterioration of the physiological functions in a physiological environment in a biological organism, such as appropriate body temperature and blood flow at a required volume to maintain the cell.

One of the most preferable methods for introducing and/or expressing cyclin and CDK in cardiomyocytes is the DNLS/CDK method previously reported by the inventors (see the patent reference 1 and the non-patent reference 5). Specifically, two types of adenovirus vectors with the D1NLS gene (cyclin D1 tagged with NLS) or the CDK4 gene integrated therein are first prepared; then, the two types of the viruses were infected into cardiomyocytes to localize the cyclin D1 protein and the CDK4 protein in the nucleus, so that the division and proliferation of cardiomyocytes generally scarcely divided or proliferated can be promoted. The contents of the patent specification and the paper are also included in the present specification.

In carrying out the invention, the method for introducing and/or expressing cyclin and CDK into cardiomyocytes may be any method as long as the method is capable of inducing the same effect as the DNLS/CDK method, with no specific limitation. For example, cyclin to be introduced and expressed in cardiomyocytes may be any cyclin capable of activating CDK4 or CDK6. Other than cyclin D1, cyclin D2- or cyclin D3 gene may be used.

Meanwhile, CDK may satisfactorily be activated by type D cyclin. As such CDK, not only CDK4 but also CDK6 may be used in accordance with the invention. Such cyclin or CDK gene has been isolated and identified from various organisms including humans. Additionally, the nucleotide sequences can be used from public DNA databases such as GenBank. Thus, a person skilled in the art can readily obtain such intended genes by designing specific primers or probes and then using general molecular biology approaches.

As the method for introducing cyclin and CDK into cardiomyocytes, physical methods such as microscopic injection may be used. From the standpoint of the introduction efficiency, gene transfection method is preferably used. Additionally, a protein molecule produced in the cytoplasm of cardiomyocytes via the expression of transfected gene is preferably transferred into the nucleus. The method therefor may be any method with no specific limitation. The method includes for example a method for tagging a nucleotide sequence encoding NLS to each of the genes. Two types of proteins generated by these genes, namely cyclin and CDK form a complex in the cytoplasm. When any one of the proteins, preferably the cyclin has the NLS sequence, accordingly, the resulting complex can be transferred into the nucleus. Currently, three types of the NLS sequences are known. Specifically, a first example is a type with almost no basic amino acids such as lysine and arginine and is for example the NLS in the nucleoprotein of influenza virus (Davy, et al., Cell 40:667, 1985). A second example is a type at a higher content of basic amino acids and is for example the NLS sequence (N-Pro-Pro-Lys-Lys-Lys-Arg-Lys-Val-C; SEQ ID NO.1) of the SV40 T antigen (Kalderon, et al., Nature 311:33, 1984). A third example is a type where basic amino acids form a cluster at an interval of about 10 amino acids and is called the NLS of the Bipartite type (Robbin, et al., Cell 64:615, 1991). The NLS for use in carrying out the invention may be any of the three types. NLS other than the three types may also be used. In view of the length of the NLS sequence, the potency of transferring a desired protein molecule into the nucleus and the ready availability of the NLS sequence gene, the NLS sequence of the SV40 T antigen is preferably used. For example, a plasmid containing the NLS sequence of the SV40 T antigen, namely pEF/myc/nus is commercially available from Invitrogen.

In accordance with the invention, the Cip/Kip family protein is a series of protein groups composing one family of the CDK-inhibitors negatively regulating the cell cycle progress and includes three molecules of $p21^{cip1}$, $p27^{Kip1}$ and $p57^{kip2}$. It is known that the Cip/Kip family protein inhibits the functions of various cyclin-CDK complexes, for example cyclin D-CDK4/CDK6 and cyclin A/E-CDK2 (see a review of Sherr & Roberts, Genes Dev. 9:1149, 1995).

Among the Cip/Kip family proteins, the function and characteristic feature of $p27^{Kip1}$ in particular is now under way of analysis. p27$^{Kip1}$ was first identified as a factor to bind to the cyclin E-CDK2 complex in a cell with the cell cycle arrest by TGF-β stimulus (Koff, et al., Cell 66:1217, 1991). It is known that p27$^{Kip1}$ is a negative cell cycle regulator responsible for the arrest of G1 phase. For example, excess expression of the p27$^{Kip1}$ protein in mammalian cells induces cell cycle arrest in the G1 phase (Polyak, et al., Cell 79:59, 1994; Toyoshima & Hunter Cell 78: 67, 1994). It is suggested that p27$^{Kip1}$ plays a significant role in the retention of the G0 phase in cells at the static phase, since p27$^{Kip1}$ is highly expressed in this stage of cells (Nourse, et al., Nature 372:570, 1994). The following researches indicate that p27$^{cip1}$ and p57$^{Kip2}$ have structures, functions and characteristic features highly similar to those of p27$^{Kip1}$ (Mainprize, et al., J. Neurooncol. 51:205, 2001; Conqueret, Trends. Cell Biol. 13 : 65, 2003). Unless otherwise stated, therefore, the term Cip/Kip (family) protein means the three types, p27$^{cip1}$, p27$^{Kip1}$ and p57$^{Kip2}$, more preferably, p27$^{Kip1}$ hereinbelow.

In carrying out the invention, the method for inhibiting the functions and actions of the Cip/Kip family protein is with no specific limitation. One example thereof is a method for suppressing the activity of the CiP/Kip protein, which includes a method of introducing neutralizing antibodies inhibiting the functions and actions of the protein or low molecular compounds or the like into the cells. Additionally, a method by which the promotion of the degradation of the Cip/Kip protein is induced is with no specific limitation. However, preferably, the method is a method of promoting the ubiquitinylation of the protein.

Ubiquitin is a polypeptide existing abundantly in all eukaryotic cells. The expression level of the Cip/Kip family protein in cells is mainly regulated with a degradation system via the ubiquitin pathway (Pagono, et al., Science 269: 682, 1995; Maki & Howley, Mol. Cell Biol. 17:355, 1997; Urano, et al., J. Biol. Chem. 274:12197, 1999). Specifically, the polyubiquitin chain is covalently bound to the Cip/Kip protein (ubiquitylated) via the functions and actions of ubiquitin-activating enzyme (E1), ubiquitin-complexing enzyme (E2) and ubiquitin ligase (E3). Subsequently, the ubiquitylated Cip/Kip protein is finally degraded with 26S proteasome. Therefore, the method for introducing a molecule promoting the ubiquitinylation of the Cip/Kip protein into cardiomyocyte is preferable in carrying out the invention. Any substance with an action promoting the ubiquitinylation of the Cip/Kip protein is satisfactory as the substance to be introduced into cardiomyocytes. Specifically, the substance includes for example pharmaceutical agents, proteins, peptides, and low molecular compounds. Preferably, nucleic acid, namely gene is used. Such gene includes genes encoding proteins composing ubiquitin-activating enzyme, ubiquitin-complexing enzyme and ubiquitin-ligase. Because it is considered that it is ubiquitin ligase that is responsible for the specificity for the ubiquitinylation of a specific target protein, preferably, genes encoding the proteins composing ubiquitin ligase (complex) are used.

As ubiquitin ligase, currently, numerous molecular species are known, including APC/C complex, VBC complex, SCF complex, Nedd4, Ufd4, Rad5, Rad18 and Parkin. Additionally, plural types of the SCF complex exist, depending on the difference in the F-box proteins contained therein as the component and are for example SCF$^{\beta TrCP}$, SCF$^{cdc4}$, SCF$^{Met30}$ and SCF$^{Grrl}$ (Patton, et al., Trends Genet. 14:236, 1998; Jackson & Eldridge, Mol. Cell 9:923, 2002). As the ubiquitin ligase (complex) involved in the ubiquitinylation of the Cip/Kip protein, ubiquitin ligase called SCF$^{skp2}$ is known. The gene encoding the component of the complex is preferably used, with no specific limitation. A gene encoding the component of ubiquitin ligase with an action to promote the ubiquitinylation of the Cip/Kip protein may also be used. It is known that in molecules composing SCF$^{skp2}$ the F-box protein called Skp2 in particular recognizes the Cip/Kip protein to bind thereto, to add the polyubiquitin chain to the protein (Carrano, et al., Nature Cell Biol . 1:193, 1999; Tsvetkov, et al., Curr. Biol. 9:661, 1999; Bornstein, et al., J. Biol. Chem. 278:26752, 2003; Kamura, et al., Proc. Natl. Acad. Sci. USA 100:10231, 2003). In carrying out the invention, therefore, the gene encoding the Skp2 protein (sometimes referred to as Skp2 gene hereinafter) is preferably used as the gene to be introduced in cardiomyocytes.

The Skp2 gene was isolated and identified in humans (Zhang, et al., Cell. 82: 915, 1995), and animals such as mouse (Nakayama, et al., EMBO J. 19:2069, 2000; Nakayama, et al., JP-A-2001-224380) and rat. The nucleotide sequence thereof is also reported. Additionally, the sequence information thereof is available in public DNA databases such as GenBank (human Skp2: U33761, AY029177; mouse Skp2: AF083215, BC003468). A person skilled in the art therefore can obtain and use the Skp2 gene by designing primers or probes specific to the Skp2 gene and using general molecular biology approaches. Skp2 genes derived from mammals such as humans, mouse or rat can bring about the same results as the Skp2 gene to be used in carrying out the invention.

In carrying out the invention, additionally, not only the Skp2 gene but also genes encoding factors promoting the ubiquitinylation and/or degradation of the Cip/Kip protein (referred to as genes promoting the degradation of the Cip/Kip protein hereinbelow) like the Skp2 gene may also be used as the genes to be introduced in cardiomyocytes. For example, genes encoding the F-box protein recognizing and binding the Cip/Kip protein, an F-box protein with 80 % or more homology, preferably 90 % or more homology with the amino acid sequence of a motif region called WD-40 repeat or leucine rich repeat considered as the substrate recognition/ binding site of Skp2, and components of ubiquitin ligase with a property to promote the ubiquitinylation of the Cip/Kip protein may also be used.

Genes encoding the proteins of any factors with an action to specifically bind to and/or interact with the p27$^{Kip1}$ protein to promote the degradation thereof without any direct involvement in the ubiquitinylation of the Cip/Kip protein may also be used for the method of the invention. Reports tell about for example Nucleoporin 50 as a nuclear membrane pore-binding protein (also referred to as Nup50, NPAP60 and p163) (Buergin, et al., EP NO. 926, 236; Mueller, et al.,

EMBO J. 19:2168, 2000; Smitherman, et al., Mol. Cell. Biol. 20: 5631, 2000; Buergin, et al., USP 6,265,562), and Jab1/CSN5 as Cop9 signalosome' (Tomoda, et al., J. Biol. Chem. 277: 2302, 2002) as such genes. It is known that when the serine residue at position 10 in p27$^{Kip1}$ is phosphorylated, p27$^{Kip1}$ binds to CRM1 protein as an extranuclear localizing transporter so that p27$^{Kip1}$ exported from nucleus and is then degraded (Ishida, et al., J Biol. Chem. 277: 14355, 2002; Connor, et al., Mol. Biol. Cell 14:201, 2003). As the specific phosphorylase of the serine residue at position 10 in the protein, KIS (Kinase interacting stathmin) has been identified (Boehm, et al., EMBO J. 21:3390, 2002). A method using the gene encoding KIS is also encompassed within the scope of carrying out the invention. In accordance with the invention, the factors or the genes associated with the factors may be used singly or in combination with a plurality thereof for the purpose of promoting the degradation of the Cip/Kip family protein

As described above, the method for carrying out the invention characteristically includes a step of introducing genes encoding cyclin and CDK, at least one of them were tagged with NLS, and the gene promoting the degradation of the Cip/Kip protein into cardiomyocytes and expressing such genes therein. Preferably, these genes are attached with a nucleic acid sequence allowing the transcription and expression of genes in mammalian cells mainly including cardiomyocytes, so-called promoter sequence in a fashion so that the transcription and expression thereof may be allowed under the regulation of the promoter. Further, the gene to be transcribed and expressed is preferably attached with polyadenylation (polyA) signal. Preferably, the promoter includes for example promoters derived from viruses such as SV (Simian virus) 40 virus, Cytomegalo virus (CMV) and Rous sarcoma virus; and β-actin promoter and EF (elongation factor) 1α promoter. Furthermore, CAG promoter (Niwa, et al., Gene 108:193, 1991) as a hybrid promoter prepared by integrating an enhancer of CMV and the polyA signal sequence of rabbit p-globin gene into chick β-actin promoter is particularly preferable.

In another mode for carrying out the invention, the promoter for use in the transcription and expression of the gene is a cardiomyocyte-specific promoter. Even in this case, the gene to be transcribed and expressed is preferably attached with poly A signal. The cardiomyocyte- specific promoter includes for example the cardiomyocyte- specific myosin light chain (Lee, et al., J. Biol. Chem. 267:15876, 1992), the cardiomyocyte-specific myosin heavy chain promoter, and the cardiomyocyte-specific cardiac ankyrin repeat protein (CARP) promoter (Cuo, et al., Development 126:4223, 1999; International publication WO 00/15821). The nucleotide sequences of these promoters are available from public DNA databases such as GenBank. By using general molecular biology approaches, gene vectors utilizing desired gene sequence can be prepared.

In accordance with the invention, a method for suppressing the expression of the gene encoding the protein (mRNA transcription) or a method for inhibiting the translation and production of the gene product may also be used instead of the method for promoting the degradation of the Cip/Kip family protein. Specifically, an oligo-nucleic acid or a derivative thereof or the like suppressing or terminating the gene encoding the Cip/Kip family protein or the gene encoding a factor capable of inducing the expression of the protein may be introduced intracellularly. In accordance with the invention, the term derivative of oligo-nucleic acid means a compound prepared by carrying out chemical modification, addition or substitution at an appropriate site of a nucleic acid for the purpose of raising the intracellular stability and incorporation efficiency of the nucleic acid. The derivative thereof includes for example phosphorothioated oligo-nucleic acid or oligo-nucleic acid prepared by substituting uridine or cytidine with 2'-fluorouridine or 2'-fluorocytidine.

As the oligo-nucleic acid available for the purpose, for example, antisense DNA, DNA encoding RNA with ribozyme activity, and decoy DNA are known. Recently, an RNA interference (referred to as RNAi) method using double-stranded RNA has been established. RNAi is a phenomenon of specific degradation of endogenous mRNA in a target gene when double-stranded RNA with the same sequence as that of the target gene or a similar sequence to that of the target gene is introduced intracellularly. It was believed at an early stage that the application of RNAi as a specific gene suppression approach to mammalian animals involved much difficulty. It was demonstrated that by using a short double-stranded RNA as an intermediate product of RNAi (referred to as short/small interfering RNA; siRNA), RNAi was also applicable to mammalian cells (Hammond, et al., Nat. Rev. Genet. 2:110, 2001; Elbashir, et al., Nature 411-494, 2002). As to reviews about siRNA, and preparation methods and methods for use thereof, see for example plural reference textbooks for example RNA Interference (RNAi) : The Nuts & Bolts of siRNA Technology (edited by Engelke, DNA Press, 2004) and RNA Interference, Editing, and Modification; Methods and Protocols (edited by Gott, Humana Press, 2004).

siRNA can readily be prepared by general polymerase chain reaction (PCR) method or chemical synthesis. It has been known that the effect of siRNA depends on the sequence feature. siRNA specific to the Cip/Kip protein gene can be prepared on the basis of the gene sequence of the gene, preferably the 300-bp sequence starting from the initiation codon. The gene for use in RNAi is not necessarily absolutely the same as the target gene. The gene for use in RNAi is with at least 70 % or more, preferably 80 % or more, more preferably 90 % or more, most preferably 95 % or more homology with the target gene. The nucleotide sequence information of the gene is available from public DNA databases such as GenBank. p27$^{Kip1}$ proteins of humans, mouse and rat are registered as Accession Nos. U10906, U10440 and D83792, respectively. Plural methods and programs for designing more effective siRNA have been reported (Chalk, et al., Biochem. Biophys. Res. Commun. 319:264, 2004; Ti-Tei, et al., Nucl. Acids Res. 32:936, 2004; Reynolds, et al., Nat. Biotechnol. 22:326, 2004). A person skilled in the art accordingly can prepare and use siRNA specific to the Cip/Kip protein gene such as the p27Kip1 gene.

siRNA prepared by the method may be used in the form of an oligo-nucleic acid or a derivative thereof. In view of for example the expression efficiency and the duration of the effect, siRNA is preferably used in a form thereof integrated in an RNA expression vector. The RNA expression vector includes for example any expression vector with a promoter capable of permitting the expression of siRNA, with no specific limitation. Pol. III promoters suitable for the expression of short RNA, particularly U6 and H1 promoters are preferably used. Additionally, tRNA promoter actively permitting the localization of transcription product in cytoplasm is preferably used. The siRNA expression vector capable of using these promoters are commercially available from Ambion, Invitrogen, TAKARA BIO and iGene.

As the method for introducing the gene or the gene vector, known methods may all be used. The method includes for example transfection methods using calcium phosphate any electric pulse, a method including sealing an intended gene into liposome and then transfecting the gene into a cell, and a method including integrating an intended gene into viral vectors such as retrovirus and adenovirus and infecting the resulting recombinant viruses into a cell. In this case, the term viral vector means a construct where an intended gene is integrated in a nucleic acid sequence with deficiency or mutation of the full length or a part of the virus DNA or RNA to permit the expression thereof.

The viral vector includes vectors derived from for example adenovirus, adeno-associated virus (AAV), retrovirus, Nippon hemagglutination virus (HVJ; Sendai virus as the other name), lentivirus, vaccinia virus, chicken pox virus, and papovavirus including SV40. Using adenovirus vector, AAV vector, HVJ vector or lentivirus vector, preferably, an efficient gene transfer and high-level expression of the transgene can be attained. The gene transfer via these virus vectors is one of the most potent methods for introducing genes into mammalian cells. Practically, the method is applicable for introducing genes into all types of human cells and a great number of non-human cells. Because infection with these viruses never depends on the cell cycle, genes can be expressed in various primary culture cell series and transformed cell series. Genes can be introduced highly efficiently even into a cell with no occurrence of DNA synthesis or cell division like cardiomyocytes. Since a great number of cells receive plural copies of recombinant DNA (RNA) after infection, the introduced gene is expressed transiently at a high level. In case of adenovirus vector and HVJ vector, for example, DNA/RNA are generally retained in cytoplasm but are hardly incorporated into nucleus. When these virus vectors are used, therefore, almost no mutagenic error occurring randomly when foreign genes are incorporated into host cell genomes hardly emerges, advantageously.

Adenovirus vector for use as one of preferable embodiments in accordance with the invention can be prepared by a method using homologous recombination in hosts such as human embryonic kidney 293 cells or Escherichia coli (Miyake, et al., Proc. Natl. Acad. Sci. USA 93:1320, 1996) and a simple *in vitro* ligation method (Mizuguchi, et al., Hum. Gene Ther. 9:2577, 1998). Adenovirus vector is one of DNA viruses with double-stranded DNA genome. Human adenoviruses of type 5 and type 2 have been researched most intensively. By deleting most of the E1 and E3 genes of these wild strain adenoviruses, virus vectors with no replication potency can be prepared, so that foreign DNA of several kb can be inserted with no adverse effects on the formation of virus particle. The recombinant adenovirus lacks the E1 gene as a transcription regulation factor. However, the adenovirus vector can express the intended gene inserted alone via a transcription unit specific to the inserted gene, with no dependence on the proliferation of a target cell or the presence or absence of another viral gene.

Persons intending the practice of the invention can see plural reference textbooks concerning reviews of adenovirus vector and other viral vectors, and methods for preparing and using such vectors. The reference textbooks include for example Gene Therapy Protocols: Methods in Molecular Medicine (edited by Robbins, Humana Press, 1997), Gene Transfer in Cardiovascular System: Experimental Approaches & Therapeutic Implications (edited by March, Kluwer Academic Publishers, 1997), Adenoviral Vectors for Gene Therapy (edited by Curiel & Douglas, Academic Press, 2002). Kits for preparing adenovirus vector are commercially available. For example, Adenovirus Expression Vector Kit (#6170) commercially available from Takara Bio is applicable to the practice of the invention. The inventors have actually reported a successful example (see Nakayama, et al., EMBO J. 19: 2069, 2000; the patent reference 1 and the non-patent reference 5 described above).

In case of intending the expression of plural genes, such as a combination of the cyclin gene and the CDK gene, and the combination with another gene promoting the degradation of the Cip/Kip protein, in accordance with the invention, these two types or three types of genes can be integrated into one virus vector for infection or can be infected in forms of individual recombinant vectors. In case of concurrent infection with plural recombinant viruses, satisfactorily, these may be simultaneously infected or may separately be infected at a given interval. As the level of viruses to be infected in accordance with the invention, a virus stock solution at for example $10^7$ to $10^{13}$ pfu/mL, more preferably $10^9$ to $10^{12}$ pfu/mL is used. For a culture cell, the virus stock solution is preferably adjusted to about 100 viruses per cell (moi = 100) for infection. The virus titer can readily be done by plaque assay.

Also described herein is that instead of (1) the cyclin gene, (2) the CDK gene, and (3) any one or a plurality of genes promoting the degradation of the Cip/Kip protein or nucleic acids inhibiting the production of the Cip/Kip family protein, a low molecular compound with the effect of expressing the gene and the same action as that of the gene may be used, specifically including for example a compound with an action similar to that of the cyclin protein, a compound with an action similar to that of the CDK protein, a compound with an action promoting the degradation of the Cip/Kip protein, or a compound with an action inhibiting the production of the Cip/Kip protein. In this case, the method for introducing the compound into cardiomyocytes is with no specific limitation. Generally, the compound is dissolved in pharmaceutically acceptable carriers such as buffered physiological saline and diluent solutions, for dosage forms such as oral administration, intravenous injections, intraperitoneal injections, transcutaneous administration, subcutaneous injections and direct injections into cardiac tissue. In case that cardiomyocytes are culture cells, further, the compound may also be added directly to the cultured medium therefor.

In a further embodiment of the invention, a pharmaceutical composition for gene therapy can be provided, the composition containing the gene vector for use in the practice of the invention, preferably viral vector, more preferably adenovirus vector or HVJ vector, AAV vector and lentivirus vector. Such pharmaceutical composition for gene therapy can be used as a pharmaceutical agent for regenerating cardiomyocytes or as a therapeutic agent for heart diseases. Any cardiac diseases may be subjects therefor, as long as the cardiac diseases involve cardiomyocytes deterioration, functional arrest or death. Specific examples thereof include cardiac infarction, ischemic cardiac diseases, congestive heart failure, hypertrophic cardiomyopathy, dilated cardiomyopathy, myocarditis, and chronic heart failure.

Any form of the pharmaceutical composition is satisfactory. The pharmaceutical composition may be formulated by routine methods. For example, the pharmaceutical composition may be in an injectable preparation form containing the gene expression vector of the invention in pharmaceutically acceptable carriers such as sterile water and buffered physiological saline, and diluent solutions. The pharmaceutically acceptable carriers may additionally contain suitable stabilizers (for example, nuclease inhibitors), chelating agents (for example, EDTA) and/or other auxiliary agents. The pharmaceutical composition containing the components may be sterilized by filtration and the like if necessary and then charged in an aseptic ampoule. Using for example an osmotic pump or an osmotic tube, the formulation may consistently be delivered to a damaged site. Herein, the dose of the pharmaceutical composition of the invention is necessarily elevated or reduced appropriately, depending on the conditions such as the age, sex, body weight and symptom of a patient and the dosage route. A person skilled in the art can appropriately determine the required dose. Generally, an adult single dose is within a range of about 1.0 $\mu$g/kg to 1.0 g/kg, preferably within a range of about 10 $\mu$g/kg to 100 mg/kg. In case of using virus vectors such as adenovirus vector, additionally, the final virus titer is preferably $10^7$ to $10^{13}$ pfu/mL, more preferably $10^9$ to $10^{12}$ pfu/mL.

In case that the gene expression vector is a non-viral vector, in particular, the pharmaceutical composition of the invention may be supplied in a complex with liposome. Via such form, the pharmaceutical composition may possibly realize a high transfection efficiency in cardiomyocytes in particular. As specific examples of liposome, a great number of lipid formulations including for example N-[2,3-(dioleyloxy)propyl]-N,N,N-trimethylammonium chloride (DOTMA) and dioleoylphosphatidylethanolamine (DOPE) have been developed. Experiments about transfection using various cell systems have been done (Banerjee, J. Biomater. Appl. 16:3, 2001; Maurer, et al., Expert Opin. Biol. Ther. 1:923, 2001). Additionally, a method using fusion-forming virus liposome with a fusion-forming envelope derived from HVJ, so-called HVJ-liposome method (Yonemitsu, et al., Int. J. Oncol. 12:1277, 1998; Kaneda, et al., Mol. Med. Today 5:298, 1999) are also effective.

The gene expression vector or a pharmaceutical composition containing the vector may satisfactorily be introduced into the whole heart of a patient with cardiac diseases. However, preferably, the gene expression vector or a pharmaceutical composition may be introduced in a limited manner into a disordered site. In accordance with the invention, the term disordered site means a site with cardiomyocytes deteriorated, functionally arrested or dead or in a vicinity thereof in individuals (humans or non-human animals: the same is true hereinbelow) or a site with an expected progress of the deterioration or functional deterioration of cardiomyocytes or the death thereof. In this case, the method for introducing the gene expression vector or a pharmaceutical composition containing the gene expression vector into a damaged site includes a method of directly injecting the gene expression vector or a pharmaceutical composition containing the same into heart post-thoracotomy, using injection syringe, and a method for injecting the vector or the pharmaceutical composition through blood tube under X-ray diaphanoscopy. The method through blood tube is preferable because the introduction of the gene can be localized just to heart by the method. In this case, the gene expression vector or a pharmaceutical composition containing the vector may be injected into blood tube to deliver the vector or the composition via blood flow into cardiomyocytes. The vector or the pharmaceutical composition may also be directly injected into the cardiac muscle layer to be in contact with cardiomyocytes. Such surgical approaches using catheter and the like are known in the field. Reference textbooks therefor include for example Gene Transfer in Cardiovascular System: Experimental Approaches & Therapeutic Implications (edited by March, Kluwer Academic Publishers, 1997), Vascular Surgery, the 5th edition (Rutherford, W.B.Saunders, 2000) and Textbook of Interventional Cardiology, the 4th edition (edited by Topol, W.B.Saunders, 2002). The catheter for use in carrying out the invention is commercially available from Boston Scientific, Edwards Life Sciences Corporation and the like.

From the cardiomyocytes proliferated by the method of the invention, highly pure cardiomyocytes can be obtained at a large scale, efficiently, by continuously using cell recovery, separation and purification processes according to known conventional methods. The cardiomyocytes obtained in such manner is called cardiomyocytes prepared in accordance with the invention hereinbelow.

Any purification method of cardiomyocytes may be used as long as the method is among known methods for cell separation and purification. Specific examples thereof include methods according to antigen-antibody reactions, for example methods with flow cytometer and magnetic beads and panning method, and cell fractionation methods by density gradient centrifugation, using carriers such as sucrose and Percol. Another screening method of cardiomyocytes is a method for selectively recovering cardiomyocytes, by preliminarily giving such artificial modification as to provide chemical tolerance and expression potency of ectopic protein to a gene of an animal or a stem cell such as ES cell as a cardiomyocyte source and then using the resulting modifications as an indicator for the screening. For example, Field and co-workers constructed a system prepared by introducing a gene cassette capable of expressing a neomycin (G418) -resistant gene into a murine ES cell under the control of myosin heavy chain-$\alpha$ promoter to differentiate the ES cells into cardiomyocytes to allow the expression of myosin heavy chain-$\alpha$ gene following the differentiation, just when the resulting cell can survive in a culture medium supplemented with G418. The researchers then report that the cell screened as the G418-resistant cell is cardiomyocytes at a probability of 99 % or more (USP 6,015,671; J. Clin. Invest. 98:216, 1996).

In an additional embodiment of the invention, the cardiomyocytes prepared in accordance with the invention is useful for the pharmacological assessment and activity evaluation of various physiologically active substances (for example, drugs) and functionally unidentified novel gene products. For example, the cardiomyocytes can be used for screening for a substance or a pharmaceutical agent associated with the functional regulation of cardiomyocytes and for a substance and a pharmaceutical agent with toxicity and a damaging property against cardiomyocytes. In a still additional embodiment, an assessment kit containing the cardiomyocytes prepared in accordance with the invention is useful for the screening.

Any type of test substances for use in the screening may be satisfactory as long as the test substances can be added to the culture system and includes for example low molecular compounds, high molecular compounds, organic compounds, inorganic compounds, protein, peptide, gene, virus, cell, liquid cell culture, and liquid microbial culture. The method for efficiently introducing the gene into the culture system includes a method of adding virus vectors such as retrovirus and adenovirus to the culture system or a method of sealing such virus vectors into liposome and the like to add the vectors to the culture system.

The assessment of test substances can be done by assaying the qualitative or quantitative change of cardiomyocyte functions. One example of assaying the viability of cardiomyocytes is as follows. The cardiomyocytes prepared in accordance with the invention is inoculated in a culture plate to an appropriate cell density to induce cell death (apoptosis) when cultured in a serum-free culture medium or to assay the survival or mortality of cardiomyocytes in a culture medium with an appropriate amount of a test substance added thereto. The method for assaying the survival or mortality of cardiomyocytes is done under visual observation or using the incorporation of a dye such as trypan blue as an indicator. As the method, otherwise, a method using dehydrogenase activity (reduction activity) as the indicators or a method using caspase activity specific to apoptotic cell or the expression of Anexin V as the indicator may also be used. Kits using the mechanism are commercially available from a great number of manufacturers such as Sigma, Clontech, and Promega for ready use.

Because the substances and the pharmaceutical agents as obtained by the screening method have an action to induce the differentiation of cardiomyocytes and an action to regulate the function, the substances and the pharmaceutical agents can be used as prophylactic agents or therapeutic agents of heart diseases, such as myocardial infarction, ischemic cardiac diseases, congestive heart failure, hypertrophic cardiomyopathy, dilated cardiomyopathy, myocarditis, and chronic heart failure. These compounds may be novel compounds or known compounds.

Additionally, the cardiomyocytes prepared in accordance with the invention may also be used as a grafting cell for regenerating cardiac muscle or for therapeutically treating heart diseases. The heart diseases include for example myocardial infarction, ischemic cardiac diseases, congestive heart failure, hypertrophic cardiomyopathy, dilated cardiomyopathy, myocarditis, and chronic heart failure. As the grafting cell, cells containing the highly pure cardiomyocyte prepared in accordance with the invention may be used in any form, such as the cells suspended in aqueous carriers such as culture media, the cells embedded in solid carriers such as biodegradable substrates or the cells processed into cardiomyocyte sheets in single layer or multiple layers (Shimizu, et al., Circ. Res. 90: e40, 2002).

The method for engrafting the cardiomyocytes as a engrafting cardiomyocyte into a damaged site includes a method of directly injecting the engrafting cardiomyocyte into heart post-thoracotomy, using injection syringe, a method for surgically incising a part of heart to engraft the cardiomyocytes and a method for injecting the cardiomyocytes through blood tube, using catheter (Murry, et al., Cold Spring Harb. Symp. Quant. Biol. 67:519, 2002; Menasche, Ann. Thorac. Surg. 75:S20, 2003; Dowell, et al., Cardiovasc. Res. 58:336, 2003). However, the method is not limited to those described above. When cardiomyocytes recovered from fetal heart is grafted into the heart of an animal with cardiac disorders, an extremely excellent therapeutic effect is exerted as reported (Menasche, Ann. Thorac. Surg. 75 : S20, 2003; Reffelmann, et al., Heart Fail. Rev. 8:201, 2003). Cardiomyocytes derived from ES cells have very similar phenotypes to those of cardiomyocytes derived from fetal heart (Maltsev, et al., Mech. Dev. 44:41, 1993; Circ. Res. 75:233, 1994). It was confirmed that in an animal experimental example where ES cell-derived cardiomyocytes were actually transplanted in an adult heart, very high biocompatibility was exerted (Klug, et al., J. Clin. Invest. 98:216, 1996). By grafting the cardiomyocytes prepared by the method of the invention into a disordered cardiac tissue in the heart diseases due to the deterioration and dissociation of cardiomyocytes in a compensatory manner, expectedly, the amelioration of the cardiac functions can be promoted.

EXAMPLES

The invention is now specifically described in the following Examples. However, the Examples below show simple illustrations of the invention but never limit the scope of the invention.

[Example 1: Preparation of recombinant adenovirus]

Adenovirus vectors, each carrying the CDK4 gene, the cyclin D1 gene with the nucleotide sequence encoding the nuclear localization signal (NLS) tagged thereto (D1NLS), or the Skp2 gene were prepared, using a recombinant adenovirus preparation kit (Adenovirus Expression Vector Kit; TaKaRa Bio).

In case of an adenovirus vector carrying the CDK4 gene, namely Ad-CDK4, a plasmid pCMV-CDK4 (supplied by Dr. Sander van den Heuvel [Massachusetts General Hospital Cancer Center; USA]; van den Heuvel, et al., Science 262:2050, 1993) was cleaved with BamHI, to prepare a murine CDK4 cDNA fragment, of which the both ends were blunted using T4 DNA polymerase. Following a protocol attached to the Adenovirus Expression Vector Kit, the blunted fragment was inserted into the SwaI site of a cosmid pAxCAwt, to prepare a cosmid pAd-CDK4. Continuously, the transfection of the cosmid together with the restriction enzyme-treated DNA-TPC (terminal peptide complex) derived from the genome DNA of human adenovirus type 5 into the 293 cells derived from human embryonic kidney cells allowed the preparation of a recombinant adenovirus, Ad-CDK4.

A plasmid carrying the D1NLS gene was constructed by conjugating the murine cyclin D1 cDNA fragment derived from pRSV-cyclin D1 (Matsushime, et al., Cell 65:701, 1991) with pEF/myc/nus (Invitrogen) - derived NLS. Specifically, the plasmid pEF/myc/nuc was digested with restriction enzymes NcoI and XhoI, to prepare a first DNA fragment containing the NLS sequence. Then, the plasmid pRSV-cyclin D1 was cleaved with a restriction enzyme NcoI, to prepare a second DNA fragment containing the cyclin D1 sequence. Then, PCR was done using the plasmid pRSV-cyclin D1 as template and also using the following two types of primers, to prepare a third DNA fragment encoding the C-terminal cyclin D1 cDNA.

5'-primer:5'-ACCCTCCATGGTAGCTGCTGGGA3' (SEQ ID NO: 2)
3'-primer:5'-TGATCTCGAGGTCGATGTCCACATCTCGCACGT-3'(SEQ ID NO: 3)

Using T4 DNA ligase, these three types of DNA fragments were ligated together, to construct a plasmid carrying a nucleotide sequence encoding SV40 T antigen-derived NLS triplicately on the side of the C terminus of the murine cyclin D1 cDNA. A DNA fragment cut out from the plasmid with restriction enzymes PmaCI and SmaI was inserted in the SwaI site of the cosmid pAxCAwt. The resulting cosmid (pAd-D1NLS) and the restriction enzyme-treated DNA TPC were transfected into the 293 cell, to prepare a recombinant adenovirus, Ad-D1NLS.

An adenovirus vector Ad-Skp2 carrying Skp2 was prepared on the basis of the murine Skp2 cDNA. The murine Skp2 cDNA was isolated from a murine thymus cDNA library (Stratagene), using the murine EST (expressed sequence tag) clone (Accession No. AA511897) registered on GenBank as a probe (Nakayama, et al., the specification of JP-A-2001-224380). Specifically, a $^{32}$p-labeled probe was prepared from the EST clone, according to general methods. Then, the probe was hybridized with a replica filter prepared on the basis of a cDNA library in a buffer at 68°C for 24 hours. The resulting hybrid was washed with a buffer containing 0.1 % SDS at 68°C. The resulting positive clone was subcloned in the pBluescript SK (Stratagene) plasmid, for nucleotide sequencing. The nucleotide sequence of the murine Skp2 cDNA as determined in such manner is registered on GenBank (Accession No. AF083215) (Nakayama, et al., EMBO J. 19:2069, 2000; Nakayama, et al., the specification of JP-A-2001-224380). Using the murine Skp2 cDNA as template and the following two types of primers, PCR was done to prepare a murine Skp2 cDNA fragment with a Flag tag sequence [N-Asp-Tyr-Lys-Arp-Asp-ASp-Asp-Lys-C; SEQ ID NO.4) at the N terminus, which was then inserted in the XhoI site of the pcDNA-3 vector (Invitrogen) to prepare a pcDNA3-Flag-Skp2 vector.

5'-primer:

5'-primer:

5'-ATACTCGAGGCCACCATGGACTACAAGGACGACGATGACAAGCATAGGAAGCACC TTCAGGAGATT-3'(SEQ ID NO:5)

3'-primer: 5'-ATACTCGAGTCATAGACAACTGGGCTTTTGCAG-3' (SEQ ID NO: 6)

A fragment containing Skp2 cDNA as obtained by the cleavage of the pcDNA3-Flag-Skp2 vector with XhoI was inserted into the SwaI site of the cosmid pAxCAwt. The resulting cosmid and a restriction enzyme-treated DNA-TPC (terminal peptide complex) derived from the genome DNA of human adenovirus type 5 were transfected into the 293 cell, to prepare a recombinant adenovirus Ad-Skp2.

The three types of recombinant adenoviruses prepared by the method described above (Ad-CDK4, Ad-D1NLS, and Ad-Skp2) are constructed in such a manner that the individual genes inserted may be expressed under the regulation of CAG promoters (CMV enhancer, chick β-actin promoter, and the polyA sequence of rabbit β-globin gene). Therefore, the inserted genes can be highly expressed in mammalian cells.

Continuously, it was intended to recover virus solutions of the individual recombinant viruses at high titers. 4 μg each of the three types of cosmids (pAd-CDK4, pAd-D1NLS and pAd-Skp2) was mixed with 2.5 5 μl of the restriction enzyme-treated DNA-TPC attached to the recombinant adenovirus preparation kit. The individual mixtures were separately transfected into the 293 cell cultured in a culture dish (diameter of 60 mm) by lipofection method using FuGENE™ 6 Transfection Reagent (Roche). On the following day, the cells were detached, and recovered cell suspensions were separately inoculated again in a culture plate (96 well) coated with collagen. After 7 to 15 days, the virus grew and the cells were killed in several wells. From each wells in which cells completely died, the culture medium was aseptically collected into a sterilized tube, freezing and thawing was repeated 6 times, and centrifuged at 500rpm for 5 minutes. The supernatants were stored as a primary virus stock solution at -80°C. 10 μl of the primary virus stock solution was infected into the 293 cells cultured in a culture plate (24 well) coated with collagen. The culture medium in a well containing killed cells in 3 to 4 days was aseptically transferred into a sterile tube, and freezing and thawing was repeated 6 times and was centrifugated at 5000 rpm for 5 minutes to recover the resulting supernatant and was stored as a secondary virus stock solution at -80°C. 15 μl of the secondary virus stock solution was infected into the 293 cells cultured in a culture flask (25 $cm^2$) coated with collagen. The culture medium after 3 to 4 days was aseptically transferred into a sterile tube, and the virus was released from cells by freezing and thawing or homogenizing cells with a sealed sonicator. The supernatant resulting from centrifugation (3000 rpm, 10 minutes, 4°C) was stored as a third virus stock solution at -80°C. 50 μl of the third virus stock solution was infected into the 293 cells cultured in a culture flask (75 $cm^2$) coated with collagen. The culture medium was aseptically transferred into a sterile tube, and the virus was released from cells by freezing and thawing or homogenizing cells with a sealed sonicator. The supernatant resulting from centrifugation (3000 rpm, 10 minutes, 4°C) was stored as a fourth virus stock solution at -80°C. The titer of the fourth virus solution was determined by plaque assay using 293 cells. The titer was constantly within a range of $10^9$ to $10^{11}$ pfu/mL, with no exception. In this disclosure, the fresh virus number to be infected per cell is expressed as multiplicity of infection (moi) hereinbelow. In other words, one virus particle infected per one cell is expressed as moi - 1.

[Example 2: Accumulation of $p27^{Kip1}$ protein in cardiomyocytes treated with DNLS/CDK]

Cardiomyocytes were isolated from a rat (Sprague-Dawley) on day 2 to day 4 from the delivery, from which a cardiomyocyte fraction was recovered by centrifugation on a Percol concentration gradient (Tamamori, et al., Am. J. Physiol. 275 : H2036, 1998). It was confirmed by immunostaining using anti-sarcomere actin antibody that 95 % or more of the cells thus recovered were cardiomyocytes. The cardiomyocytes from the newborn rat were suspended in an Eagle minimum essential culture medium (Flow Laboratories) supplemented with 5 % bovine fetus serum (FBS; Flow Laboratories) and then inoculated in a culture dish for culturing at 37°C in a carbon dioxide incubator for 24 hours. On the next day, the culture medium was exchanged with serum-free Eagle minimum essential culture medium for another 24-hr culturing. Subsequently, the recombinant virus Ad-D1NLS (mom = 10 to 100) and Ad-CDK4 (moi = 100) as prepared in Example 1 were added to the culture medium, for 48 hours culturing. The procedure for the infection and transfection of cardiomyocytes with the recombinant viruses Ad-D1NLS and Ad-CDK4 to express the cyclin D1 protein and the CDK4 protein in the nucleus of cardiomyocytes is sometimes referred to as stimulation with D1NLS+CDK4 or treatment with D1NLS + CDK4 hereinbelow. As a control, the same experiment was done, using REF52 cells as a fibroblast cell line.

The expression of $p27^{Kip1}$ protein in the cardiomyocytes transfected with the D1NLS and CDK4 genes was examined by Western blotting. The cells transfected with Ad-D1NLS and Ad-CDK4 viruses were washed with ice-cold phosphate buffered saline (PBS) and then scraped with a cell scraper, for centrifugation to discard the resulting supernatant. The resulting precipitate was once again rinsed with a small volume of PBS, and was then transferred into a 1.5-mL Eppendorf tube, to which ice-cold Buffer A (10 mM HEPES, pH 7.9, 1.5 mM $MgCl_2$, 10 mM KCl, 0.5 mM DTT) was added. Then, the resulting mixture was agitated and left to stand alone on ice for 10 minutes. Subsequently, NONIDET P-40 was added to the mixture to a final concentration of 0.2 % for agitation and subsequently allowing the mixture to stand alone on ice for 5 minutes. To a precipitate resulting from another centrifugation (5, 000 rpm, 5 minutes) was added an equal volume of Buffer C (20 mM HEPES, pH 7.9, 25 % glycerol, 0.42 M NaCl, 1.5 mM $MgCl_2$, 0.2 mM EDTA) for agitation. The resulting mixture was left to stand alone on ice for 30 minutes, for centrifugation (15,000 rpm, 10 minutes) to recover the supernatant, which was used as nuclear protein fraction. Immediately before use, furthermore, 1 mM DTT, 1 mM PMSF, 1 μg/ml aprotinin, 1 μg/ml leupeptin, and 1 μg/ml pepstatin (all from Sigma) were added to the Buffer A and Buffer C described above.

The nuclear protein obtained in such manner was adjusted to a volume containing $1 \times 10^6$ cells per one sample, electrophoresed on a gel for SDS-PAGE, transferred onto a nitrocellulose membrane and analyzed by Western blotting. Specifically, the nuclear protein reacted with an anti-cyclin D1 antibody (Oncogene Science: Ab-3) or anti-$p27^{Kip1}$ antibody (Santa Cruz; sc-528) as a primary antibody and subsequently reacted with horseradish peroxidase-labeled anti-mouse Ig antibody (Amersham Life Science; NA931) or anti-rabbit Ig antibody (Amersham Life Science; NA934) as a second antibody to detect the presence of the antigen bound to the antibody, using a chemiluminescence kit (Amersham Life Science; RPN2109).

The results are shown in Fig.1. Like the previous reports (Nakayama, et al., EMBO J. 19: 2069, 2000; the patent reference 1 and the non-patent reference 5), it was confirmed that the transfection of cardiomyocytes with the D1NLS and CDK genes increased the expression levels of the cyclin D1 protein and the CDK4 protein in the nucleus of cardiomyocytes. Then, the expression of the $p27^{Kip1}$ protein was more intensely induced, as the expression level of the cyclin D1 protein was increased. In the fibroblasts (REF52 cells), meanwhile, the increase of the expression of the cyclin D1 protein rather reduced the expression level of the $p27^{Kip1}$ protein. The difference in the performance of the $p27^{Kip1}$ protein under the stimulation with D1NLS + CDK4 was observed in the two types of the cells.

Continuously, the expression and localization of the $p27^{Kip1}$ protein in cardiomyocytes was examined by immunocytochemical staining method. Like the method described above, cardiomyocytes transfected with Ad-D1NLS and Ad-CDk4 viruses (moi = 100) were fixed with 70 % ethanol 48 hours after the virus infection. Then, the anti-$p27^{Kip1}$ antibody (the same as described above) (diluted to 1:1000) and an anti-sarcomeric actin antibody (DAKO; M0874) (diluted to 1:100) reacted with the resulting cardiomyocytes, to stain the cardiomyocytes, using Alexa Fluor™-labeled antibodies (Alexa-488 and Alexa-568; Molecular Probes) (both diluted to 1:200). Further, the cellular nucleus was stained with 4',6-diamidino-2-phenylindole (DAPI) solution (1 μg/mL). The images stained with these antibodies and the dye were observed with a fluorescent microscope (laser scanning co-focus image system; Zeiss LSM510).

The results are shown in Fig.2. The expression of $p27^{Kip1}$ was observed in the nucleus of intact cardiomyocytes (cells positive with sarcomeric actin in the figure), but it was not so intense. Intense expression and nuclear accumulation of $p27^{Kip1}$ was confirmed in the cardiomyocytes transfected with the D1NLS and CDK4 genes for expression. The results in Figs. 1 and 2 suggested that the enforced expression of the D1NLS and CDK4 genes in cardiomyocytes allowed the accumulation of $p27^{Kip1}$ protein suppr'essing the progress of the cell cycle in the nucleus of cardiomyocytes, so that the division and proliferation potencies of the cardiomyocytes as induced by the stimulation with D1NLS + CDK4 might possibly be suppressed.

So as to examine whether the increase of the $p27^{Kip1}$ expression level under stimulation with D1NLS + CDK4 was regulated at the transcription level, Northern blotting analysis was done. Even in any of cardiomyocytes and fibroblasts, however, the enforced expression of D1NLS and CDK4 genes had no influence on the expression level of $p27^{Kip1}$ mRNA. It is known that the expression protein level of $p27^{Kip1}$ in proliferating cells is suppressed with the ubiquitin-proteasome degradation system (Carrano, et al., Nature Cell Biol. 1:193, 1999; Tsvetkov, et al., Curr. Biol. 9: 661, 1999) . Thus, examination was done as to whether or not the expression level of the $p27^{Kip1}$ protein was adjusted even in cardiomyocytes with the proteasome degradation system. After the cardiomyocytes prepared by the method were cultured in a state of serum starvation for 24 hours, Ad-D1NLS and Ad-CDK4 (moi = 100) were added to the culture medium for 48 hours culturing. In different groups, cardiomyocytes were cultured in a state of serum starvation for 48 hours. Then, FBS at a volume to 10 % was added to the culture medium, for 24 hours culturing. Then, lactastatin (20 μM) (Santa Cruz) as a proteasome inhibitor was simultaneously added to one of the groups, while the inhibitor was not added to the other group. The expression level of the $p27^{Kip1}$ protein in each of the groups was examined by Western blotting.

Consequently, a distinct elevation of the expression level of the $p27^{Kip1}$ protein was confirmed (Fig.3) as in Fig.1. Alternatively, the expression level of the $p27^{Kip1}$ protein was reduced under stimulation with FBS. The inventors indicated in the previous reports that the cyclin D1 protein and the CDK4 protein were hardly located into the nucleus of cardiomyocytes although these molecules were increasingly expressed on FBS stimulation, so that no cell cycle was activated (see the patent reference 1 and the non-patent reference 5). It was suggested that the increase of the amount of the $p27^{Kip1}$ protein in cardiomyocytes was specific to the stimulation for the nuclear transfer of the cyclin D1 protein and/or the CDK4 protein. In case that the function and action of proteasome was suppressed with lactastatin treatment, alternatively, no difference in the expression level of the p27$^{Kip1}$ protein was observed among the no-addition group, the FBS-treated group and the group treated with D1NLS + CDK4. The results mean that the function and action of the p27$^{Kip1}$ protein are inhibited in the group treated with D1NLS + CDK4 although the p27$^{Kip1}$ protein is constantly degraded in the no-addition group or the FBS-treated group.

So as to examine the ubiquitinylation potency of the p27$^{Kip1}$ protein in cardiomyocytes, continuously, *in vitro* ubiquitinylation assay was done. Details of the experimental method are according to the method described in the report papers by the inventors (Nakayama, et al., EMBO J. 19: 2069, 2000; Nakayama, et al., JP-A-2001-224380; Hara, et al., J. Biol. Chem. 276:48937, 2001; Ishida, et al., J. Biol. Chem. 277:14355, 2002). The cardiomyocytes prepared by the aforementioned method were cultured in a state of serum starvation for 24 hours, which were subsequently cultured for 48 hours in the culture medium supplemented with Ad-D1LNS and Ad-CDK4 (moi = 100). In a different group, the cardiomyocytes were cultured in a state of serum starvation for 48 hours, which were then cultured for 24 hours in the culture medium supplemented with FBS to an amount of 10 %. As a control, REF52 cells cultured in a 10 % FBS-supplemented culture medium were used. After these cells were rinsed with ice-cold PBS, the cells were scraped with a cell scraper and then centrifuged, to discard the supernatant. To the resulting precipitate was added a 2-fold volume of the Buffer C (described above) supplemented with 0.5 % NONIDET F-40. Then, the resulting mixture was agitated and left to stand alone on ice for 30 minutes, for ultrasonic disruption. Subsequently, the resulting mixture was centrifuged (15,000 rpm, 20 minutes) to recover the supernatant, which was used as a cell extract.

The recombinant p27$^{Kip1}$ protein for use as a substrate for ubiquitinylation was prepared by an *in vitro* translation system using rabbit reticulocyte lysate. Specifically, the *in vitro* transcription and translation was done, using a commercially available *in vitro* transcription translation kit (TnT coupled Reticulocyte Lysate System; Promega) and using mouse p27$^{Kip1}$ cDNA with a FLAG tag sequence tagged thereto as template according to the attached protocol. The recombinant p27$^{Kip1}$ protein thus prepared and each reticulocyte lysate sample (20 to 40 μg as protein amount) were prepared together with the mouse E1 protein (50 μg/mL), mouse E2/Ubs5 protein (100 μg/mL), and GST-Ub protein (4 mg/mL) (all from Calbiochem) in a reaction solution of a final 10 - μl volume (4 mM Tris-HCl (pH 7.5), 6 mM NaCl, 5 mM $MgCl_2$, 0.1 mM DTT, 0.1 mg/mL creatine phosphokinase, 10 mM phosphocreatine, 1.5 mM ATP), for reacting them together at 26°C for 30 minutes. Subsequently, the sample was electrophoresed on a gel for SDS-PAGE and transferred onto a nitrocellulose membrane for Western blotting analysis. Specifically, reaction with a first antibody anti-Flag antibody (Sigma; F-3165) or anti-GST antibody (Santa Cruz; sc-138) (both diluted to 1:1000) and reaction with a second antibody horseradish peroxidase-labeled anti-mouse Ig (as mentioned above) (diluted to 1:1000) enabled the detection of the presence of the antigen bound to such antibody, using a chemiluminescent kit (as described above).

The results are shown in Fig.4. In the p27$^{Kip1}$ protein *in vitro* ubiquitinylated using a cell extract of fibroblasts during proliferation (on stimulation with FBS) (REF in the figure) as a positive control, the presence of the *in vitro* ubiquitinylated p27$^{Kip1}$ protein could clearly be detected, by using the antibody recognizing the Flag peptide tagged to the recombinant p27$^{Kip1}$ protein (IB: Flag in the figure) and a specific antibody (IB: GST in the figure) recognizing the GST protein added to the recombinant ubiquitin protein. When a cell extract prepared from cardiomyocytes stimulated with FBS was used, however, just weak ubiquitinylated p27$^{Kip1}$ protein was observed, and a very vague band of the ubiquitinylated p27$^{Kip1}$ protein was only detected in cardiomyocytes transfected with D1NLS and CDK4 genes. The results shown in Figs. 3 and 4 strongly suggest that the ubiquitinylation of the p27$^{Kip1}$ protein was significantly suppressed in cardiomyocytes under stimulation with D1NLS + CDK4 so that proteasome degradation hardly occurred, consequently involving the nuclear accumulation of the p27$^{Kip1}$ protein.

[Example 3: Decrease of p27$^{Kip1}$ protein accumulated in cardiomyocytes by enforced Skp2 expression]

It is known that the p27$^{Kip1}$ protein is ubiquitinylated in general proliferating cells with a ubiquitin ligase containing Skp2 as an F-box protein, namely the SCF$^{Skp2}$ complex so that the p27$^{Kip1}$ protein is degraded with proteasome (Carrano, et al., Nature Cell Biol. 1:193, 1999; Tsverkiv, et al., Curr. Biol. 9:661, 1999). Therefore, the expression of the Skp2 protein in cardiomyocytes was examined by Western blotting. Methods for cardiomyocyte preparation, transfection with Ad-D1NLS and Ad-CDK4, nuclear protein preparation, Western blotting and the like were the same methods as described above. So as to detect the Skp2 protein, the anti-Skp2 antibody was used.

The results are shown in Fig.5. The expression of the Skp2 protein in fibroblasts (REF52 cells) used as a control cell was significantly activated under FBS stimulation and stimulation with D1NLS and CDK4 promoting the proliferation. However, almost no induction of the expression of the Skp2 protein was observed in cardiomyocytes, while in the cardiomyocytes transfected with D1NLS and CDK4 genes, only a trace amount of the Skp2 protein could be detected. The results suggest a possibility of the suppression of the degradation of the p27$^{Kip1}$ protein accumulated under stimulation with D1NLS + CDK4 because of no occurrence of the induction of the expression of Skp2 in cardiomyocytes.

Accordingly, examination was done about the effect of the Skp2 gene on the expression of the p27$^{Kip1}$ protein via the co-expression of the Skp2 gene in cardiomyocytes transfected with D1NLS and CDK4 genes. Cardiomyocytes were transfected with Ad-D1NLS (moi - 100), Ad-CDK4 (moi = 100) and Ad-Skp2 (moi = 50, 100) prepared in Example 1 for 48 hours culturing, to analyze the expression of cyclin D1, Skp2 and p27$^{Kip1}$ protein by Western blotting. Methods for cardiomyocyte preparation, nuclear protein preparation, Western blotting analysis and the like were the same methods as described above.

Three genes for D1NLS, CDK4 and Skp2 were concurrently expressed in cardiomyocytes. The results are shown in Fig.6. When the D1NLS and CDK4 genes were transfected in cardiomyocytes, the expression of the p27$^{Kip1}$ protein was strongly induced as in the previous experiments (Figs. 1 and 3). In that case, transfection of Ad-Skp2 into cardiomyocytes to subsequently allow the concurrent expression of the Skp2 gene significantly reduced the expression level of the p27$^{Kip1}$ protein. In cardiomyocytes infected with a high concentration (moi = 100) of Ad-Skp2, almost no expression of the p27$^{Kip1}$ protein was observed.

It was further confirmed using an immunocytostaining method as shown in Fig.7 that the accumulation of the p27$^{Kip1}$ protein in cardiomyocytes transfected with the D1NLS and CDK4 genes was reduced by the co-expression of the Skp2 gene. The immunocytostaining method is the same method as in the experiment described above (Fig. 2). As is shown in Fig.2, intense expression of p27$^{Kip1}$ and nuclear accumulation thereof were observed in cardiomyocytes transfected with D1NLS and CDK4 genes. When the Skp2 gene was co-expressed, alternatively, significant reduction of the p27$^{Kip1}$ protein was observed in the nucleus of the cell. When cardiomyocytes with D1NLS, CDK4 and Skp2 genes concurrently expressed therein were treated with lactastatin, the expression level of the p27$^{Kip1}$ protein was resumed to almost the same level as the level during stimulation with D1NLS and CDK4. Thus, it was confirmed that the reduction of the p27$^{Kip1}$ protein by the enforced expression of the Skp2 protein was mediated by the ubiquitin-proteasome degradation system.

[Example 4: Effect of enforced Skp2 expression on the promotion of the proliferation of cardiomyocytes]

By allowing Ad-D1NLS, Ad-CDK4 and Ad-Skp2 (individually moi = 100) to transfect cardiomyocytes and subsequently counting the cell number periodically, the influence of the introduction of the Skp2 gene on the proliferation potency of cardiomyocytes was examined. Like the results in the previous reports (see the patent reference 1 and the non-patent reference 5), the cell number of the cardiomyocytes with D1NLS and CDK4 genes expressed therein was increased about 3 fold on day 7 post-culturing (Fig. 8). It was alternatively confirmed that the cell number of cardiomyocytes with the three genes namely D1NLS, CDK4 and Skp2 genes expressed therein was increased 5 fold or more. Almost no increase of the cell numbers of cardiomyocytes infected with control vector and cardiomyocytes infected with Ad-Skp2 alone as negative controls was observed. The aforementioned results apparently indicate that Skp2 more significantly may promote the proliferation potency of the cardiomyocytes as promoted on stimulation with D1NLS + CDK4.

[Example 5: Effect of p27 siRNA treatment on promotion of proliferation of cardiomyocytes}

So as to more clearly demonstrate the influence of the p27$^{Kip1}$ protein in cardiomyocytes treated with D1NLS and CDK4, siRNA specific to the p27$^{Kip1}$ gene (referred to as 'p27 siRNA' hereinafter) was expressed in cardiomyocytes, to examine the effect.

So as to construct a vector for expressing p27 siRNA, target sequences were determined on the basis of the nucleotide sequence information of rat p27$^{Kip1}$ cDNA (accession No.: D83792 in GenBank) while an oligo DNA for use in constructing a vector expressing the siRNA corresponding to the sequence was designed and prepared. As the target sequences, the following three types were selected from rat p27$^{Kip1}$ cDNA :

nucleotide Nos: 830-847 (5'-GGCAGAAGATTCTTCTTC-3': SEQ ID NO. 7) ;
nucleotide Nos: 532-550 (5'-AGCGCAAGTGGAATTTCGA-3': SEQ ID NO. : 8); and
nucleotide Nos: 372-390 (5'-GTGAGAGTGTCTAACGGGA-3': SEQ ID NO. 9) siRNAs based on SEQ ID NOS: 7 through 9 are referred to as siRNA #1, #4 and #6, respectively, hereinbelow. The inserted oligo DNA sequences used in constructing vectors expressing siRNA #1, #4 and #6 are as follows.

siRNA #1: 5'-CACCGGTAGGAGGTTCTTCTTCAACGTGTG CTGTCCGTTGAAGAAGAATC TTCTGCCTTT TT-3' (SEQ ID NO.: 10) and 5'-GCATAAAAAG GCAGAAGATT CTTCTTCAAC GGACAGCACA CGTTGAAGAA GAACCTCCTACC-3'(SEQ ID NO: 11).

siRNA #4: 5'-CACCAGTGTA AGTGGAGTTT CGAACGTGTG CTGTCCGTTC GAAATTCCAC TTGCGCTTTT TT-3' (SEQ ID NO.: 12) and 5'-GCATAAAAAAGCGCAAGTGG AATTTCGAAC GGACAGCACA CGTTCGAAAC TCCACTTACA CT-3'(SEQ ID NO: 13).

siRNA #6: 5'-CACCGTGGGA GTGTTTAATG GGAACGTGTG CTGTCCGTTC CCGTTAGACA CTCTCACTTT TT-3' (SEQ ID NO.: 14) and 5'-GCATAAAAAG TGAGAGTGTC TAACGGGAAC GGACAGCACA CGTTCCCATT AAACACTCCC AC-3' (SEQ ID NO: 15).

Three groups of oligo-DNAs each containing the siRNA target sequence were annealed and then inserted into the BsmMI site of an RNA expression vector (pcPURU6 β icassette, iGENE). The vector is in a construction such that the transcription of RNA corresponding to the inserted genes may start under control of human U6 promoter to highly express the intended RNA in mammalian cells. Then, the vector was cleaved with EcoRI and HindIII, to purify the resulting fragment containing the U6 promoter and the inserted gene sequences. After the end was blunt-ended using T4 DNA polymerase, the fragment was inserted into the SwaI site of cosmid pAxcwit (TAKARA BIO). Continuous transfection of 293 cells with the cosmid and restriction enzyme-treated DNA-TPC derived from the genome DNA of human adenovirus of type 5 enabled the preparation of recombinant adenoviruses expressing $p27^{Kip1}$ siRNA (Ad-p27 siRNA-#1, #4, #6). Continuously, a virus solution with a high titer was recovered from the virus vector by the same method as in Example 1.

First, p27 siRNA was co-expressed in cardiomyocytes transfected with D1NLS and CDK4 genes, to examine the effect on the expression of the $p27^{Kip1}$ protein by Western blotting. Specifically, Ad-D1NLS, Ad-CDK4 and Ad-p27 siRNA (individually moi = 100) were transfected into cardiomyocytes for 48-hr culturing, to analyze the expression of the $p27^{Kip1}$ protein by Western blotting Methods for cardiomyocyte preparation, nuclear protein preparation, Western blotting analysis and the like are the same as the methods described above (Examples 1 and 3).

The results are shown in Fig.9. When D1NLS and CDK4 genes were transfected into cardiomyocytes, the expression of the $p27^{Kip1}$ protein was strongly induced as in the experiments described above (Figs. 1, 3 and 7). By expressing p27 siRNAs (#1, #4, #6) in cardiomyocytes, the production of the $p27^{Kip1}$ protein was greatly reduced. Almost no expression of the $p27^{Kip1}$ protein was observed with p27 siRNA-#6 in particular. The expression of p27 siRNA exerts a suppressive effect specific to the $p27^{Kip1}$ protein but never influences the intracellular contents of the $p21^{Kip1}$ protein, the cyclin D1 protein or sarcomeric actin. Even in the results of the immunocytostaining, almost no accumulation of the $p27^{Kip1}$ protein was observed in the nucleus of cardiomyocytes through the expression of p27 siRNA.

Continuously, Ad-D1NLS, Ad-CDK4 and Ad-p27 siRNA-#6 (individually moi = 100) were transfected into cardiomyocytes, to subsequently count the cell number periodically. Like the results in Example 4, the cell number of cardiomyocytes with D1NLS and CDK4 genes expressed therein was increased about 3 fold on day 7 post-culturing (Fig. 10). Meanwhile, it was verified that the cell number of cardiomyocytes with the three genes namely D1NLS, CDK4 and p27 siRNA genes expressed therein was significantly increased. Almost no increase of the cell numbers of cardiomyocytes infected with LacZ expression virus and cardiomyocytes infected with Ad-p27 siRNA alone as negative controls was observed. The aforementioned results apparently indicate that the expression of p27 siRNA more significantly may promote the proliferation potency of the cardiomyocytes as promoted on stimulation with D1NLS + CDK4.

[Example 6: Effect of enforced Skp2 gene expression on cardiac therapy]

So as to confirm that the effect of the enforced expression of D1NLS + CDK4 + Skp2 genes on the proliferation of cardiomyocytes had a therapeutic effect on damaged cardiomyocytes, examination was done, using a rat myocardial ischemia and reperfusion model. The model was prepared according to the method by Dairaku, et al. (Circ. J. 66:411, 2002). A male Wistar rat (age 8 weeks) was anesthetized via intraperitoneal administration of sodium pentobarbital (Nembutal: Dainippon Pharmaceutical Co., Ltd.) (55 mg/kg). Subsequently, the rat received thoracotomy under artificial respiration, to expose the heart. Continuously, left coronary artery was ligated with a suturing thread with a needle No. 5-0. Then, the rat was left to stand for 30 minutes. The ligature was released, to allow blood flow to be reperfused (reperfusion). As a control, an animal treated only with threading the coronary artery was used as a sham operation group (referred to as Sham group hereinafter).

. The adenovirus vector was introduced into the heart during a period of 25 to 30 minutes post-ischemia. A high-titered adenovirus solution ($1 \times 10^9$ pfu/mL) of the same lot as prepared and used in the above Example was directly injected into the cardiac muscle layer in the ischemic center and in the periphery thereof at 50 μl each per one site in total of 5 sites (the total volume of 250 μl), using a 30G injection needle.

So as to examine the effect of the treatment with D1NLS + CDK4, an animal group injected with a mixture solution of the three types of adenoviruses, namely Ad-D1NLS ($1 \times 10^9$ pfu), Ad-CDK4 ($1 \times 10^8$ pfu) and Ad-LAcZ ($1 \times 10^9$ pfu) is referred to as 'D1NLS group' hereinbelow. So as to examine the effect of the treatment with D1NLS + LCDK4 + Skp2, an animal group injected with a mixture solution of the three types of adenoviruses, namely Ad-D1NLS ($1 \times 10^9$ pfu), Ad-CDK4 ($1 \times 10^8$ pfu) and Ad·Skp2 ($1 \times 10^9$ pfu) is referred to as 'Skp2 group' hereinbelow. As a negative control group, an animal group injected with Ad-LacZ ($2 \times 10^9$ pfu) was arranged (referred to as 'Cont group' hereinafter). No injection of any adenovirus solution was done in the Sham group. After closing the chest, the rat was awakened from anesthesia, and then fed under general feeding conditions for 6 weeks.

As an indicator of the necrosis of cardiac muscle following ischemia and disordered reperfusion, the cardiac muscle troponin T (cTnT) value in plasma was assayed (O'brien, et al., Lab. Anim. Sci. 47:486, 1997; Morimoto, et al., J. Pharmacol. Sci. 91:151 [Suppl. 1], 2003). 2 hours after reperfusion, blood was drawn out from the fundus and centrifuged to recover plasma. The cTnT value was assayed, using a cardiac reader (Roche Diagnostics). Consequently, it was observed that the cTnT value after ischemia and reperfusion was greatly increased in the Cont group, the D1NLS group and the Skp2 group, compared with the Sham group (Sham group: $0.2 \pm 0.0$ ng/mL, Cont group: $8.8 \pm 0.5$ ng/mL, D1NLS group: 9.1 ± 0.7 ng/mL, Skp2 group: 9.9 ± 1.3 ng/mL). No difference in the cTnT value among the three groups except the Sham group was observed, suggesting that a similar level of the necrosis of cardiac muscle was induced.

6 weeks after reperfusion, cardiac functions were measured by tomography (B mode method) and the M mode method displaying tomography over time, using an ultrasonography apparatus(power Vision 8000: Toshiba Medical). Ketamine (Ketaral: Sankyo) and xylazine (Sigma) were intraperitoneally administered to a rat for anesthesia. Using a linear probe of 15 MHz, the left ventricular short-axis papillary muscle was measured by the M mode method, to measure the left ventricular end-diastolic dimension and the left ventricular end-systolic dimension to calculate the fractional shortening of left ventricular inner diameter (FS).

$$\text{Fractional Shortening of left ventricular inner diameter (FS)} = (\text{end-diastolic dimension} - \text{end-systolic dimension}) / \text{end-diastolic dimension} \times 100\ (\%)$$

By measuring left ventricular longitudinal axis by the B mode method, left ventricular end-diastolic area and end-systolic area were measured, to calculate the fractional area change of left ventricular (FAC), which was used as an indicator of the systolic function.

$$\text{Fractional Area Change of left ventricular (FAC)} = (\text{end-diastolic area} - \text{end-systolic area}) / \text{end-diastolic area} \times 100\ (\%)$$

Using a 10 MHz sector probe, additionally, left ventricular blood flow influx was measured at early diastolic stage (E) and atrium systolic stage (A) by Doppler method, to calculate E/A value, which was used as an indicator of diastolic function. The aforementioned measurements were done in a blind test manner so that a test practitioner could not figure out the contents of the treatment of each animal.

6 weeks after myocardial ischemia and reperfusion, cardiac functions were measured by the methods described above. In the Cont group compared with the Sham group, the fractional shortening (FS) and the fractional area change (FAC) were at very small values while the left ventricular blood flow influx ratio at the early diastolic stage and the systolic atrium stage (E/A) was at a large value. Thus, it was observed that the systolic function and diastolic function were apparently deteriorated. In the D1NLS group or the Skp2 group, in contrast, FS and FAC were at larger values than in the Cont group. A more apparent amelioration effect was observed in terms of the E/A value. Compared with the Cont group, the E/A value in the Skp2 group was at a significantly small value. The E/A value in the D1NLS group was smaller than in the Cont group, but no statistically significant difference was observed between them (Sham group: 2.30 ± 0.25, Cont group: 5.49 ± 0.86, D1NLS group: 3.69 ± 0.68, Skp2 group: 3.44 ± 0.57 : n = 9 to 12).

Continuously, cardiac hemodynamics was analyzed on the next day of the cardiac ultrasonography test. After sodium pentobarbital was intraperitoneally given to a rat for anesthesia, a microchip pressure transducer-equipped catheter (SPC-320: Millar Instruments) was inserted into the left ventricle from the right carotid artery, to measure the maximum differential rate (dP/dt max) of left ventricle pressure (LVP) as an indicator of left ventricular systolic potency and measure dp/dt/P max obtained by dividing dP/dt max with LVP simultaneously measured as an indicator of the left ventricular diastolic potency. Furthermore, the minimum differential rate (dP/dt min) of LVP as an indicator of left ventricular diastolic potency and left ventricular end-diastolic pressure (LVEDP) was measured. The aforementioned measurements were done in a blind test manner so that a test person could not identify the contents of the treatment of each animal. The results are shown in Table 1.

[Table 1]

| Experimental group | Sham | Cont | D1NLS | Skp2 |
|---|---|---|---|---|
| Number | 9 | 10 | 12 | 10 |

(continued)

| Experimental group | Sham | Cont | D1NLS | Skp2 |
|---|---|---|---|---|
| LVEDP (mmHg) | 3.4 ± 1.2 | 13.3 ± 2.5※ | 9.5 ± 2.3 | 8.5 ± 1.7 |
| LV dP/dt max (mmHg/sec) | 7457 ± 308 | 5572 ± 230※ | 6022 ± 264※ | 6183 ± 250※ |
| LV dP/dt min (-mmHg/sec) | 6492 ± 312 | 3631 ± 182※ | 4047 ± 270※ | 4449 ± 248※ # |
| LV dP/dt/P max (1/sec) | 75 ± 5 | 40 ± 3※ | 51 ± 5※ | 54 ± 4※ # |

※ : p < 0.05 vs. Sham group
#: p < 0.05 vs. Cont group

Compared with the Sham group, the left ventricular end-diastolic pressure (LVEDP) in the Cont group was at a significantly high value. Additionally, the maximum differential rate (dP/dt max), the minimum differential rate (dP/dt min) or dP/dt/P max of the left ventricular pressure (LVP) was at a significantly small value. The results indicate that the left ventricular systolic function and diastolic function in the animal were deteriorated. In the D1NLS group, the three indicators of dP/dt max, dp/dt min and dP/dt/P max were at larger values than in the Cont group. The individual indicators were all at larger values in the Skp2 group than in the D1NLS group, while the dp/dt min and dP/dt/P max were at significantly larger values than in the Cont group.

After cardiac hemodynamics was counted, the lung was resected to weigh the wet weight (Fig. 11). Compared with the Sham group, the lung weight in the Cont group was significantly larger. This suggests a possible occurrence of lung congestion. Compared with the Cont group, lung weight was smaller in the D1NLS group while the lung weight in the Skp2 group was at a significantly small value, suggesting the amelioration of lung congestion.

Using the resected heart after the measurement of cardiac functions, a passive (left ventricle) pressure-volume curve was prepared. The fundamental method was according to the report of Pfeffer, et al. (Circ. Res. 57:84, 1985). Specifically, heparin (Novo-heparin: Aventis Pharma) and saturated potassium chloride were given from the tail vein of the rat, to induce cardiac arrest at the diastolic stage. Immediately thereafter, the heart was resected. Through the aorta, a double-lumen catheter (DP-8: Natsume Seisakusho) was inserted into the left ventricle. Connecting one of the tubes to the pressure transducer to measure the left ventricular pressure, physiological saline was injected at a rate of 0.72 mL/min from the other tube, to record a pressure-volume curve on the chart. One resected heart was tested in a triplicate manner, to calculate the average . Based on the pressure-volume curve of an individual animal, the volume corresponding to the left ventricular end-diastolic pressure measured with the microchip pressure transducer-equipped catheter was calculated and then corrected on the weight basis, to calculate the left ventricular end-diastolic volume index (LVEDVI).

The results are shown in Fig.12. Compared with the Sham group, the passive pressure-volume curve in the Cont group was significantly shifted on the right side, as observed. The results indicate a possibility of the progress in left ventricular remodeling such as the increase of left ventricular volume and the thinning of infarct area after cardiac ischemia and reperfusion. Compared with the Cont group, the rightward shift of the passive pressure-volume curve was reduced in the D1NLS group. Compared with not only the Cont group but also the D1NLS group, the rightward shift of the pressure-volume curve in the Skp2 group was significantly reduced. While the left ventricular end-diastolic volume index (LVEDVI) was at a large value in the Cont group, the index was at a smaller value in the D1NLS group than in the Cont group. In the Skp2 group, further, the index was at a smaller value than in the D1NLS group and was at a significantly smaller value than in the Cont group (Sham group: 0.80 ± 0.12 mL/kg, LacZ group: 2.18 ± 0.16 mL/kg, D1NLS/CDK4 group: 1.72 ± 0.19 mL/kg, Skp2 group: 1.54 ± 0.17 mL/kg; n = 9 to 12).

Finally, the level of cardiac infarction in the heart 6 weeks post-ischemia and reperfusion was examined. The resected heart was fixed in a 10 % neutrally buffered formalin solution, embedded in paraffin, prepared into 6 sections at an interval of 2 mm in each sample along the cross-sectional direction, and stained with Masson Trichrome to visualize the area with cardiac infarction. Then, the infarct area was measured using an image analysis software (Lumina vision: Mitsuya Shoji). With reference of the report of Jain, et al. (Circulation 103:1920, 2001), the infarct area was measured by measuring the whole peripheral length of the inner membrane of left ventricle, the whole peripheral length of the outer membrane thereof, the peripheral length of the cicatrisation on the side of the inner membrane of left ventricle and the peripheral length of the cicatrisation on the side of the outer membrane thereof to calculate the infarct area according to the following formula.

Infarct area = [(the peripheral length of the cicatrisation on the side of the inner membrane of left ventricle + the peripheral length of the cicatrisation on the side of the outer membrane thereof)/ (the whole peripheral length of the inner membrane + the whole peripheral length of the outer membrane)] × 100 (%)

The results are shown in Fig.13. Compared with the Cont group, the infarct area in the D1NLS group was greatly reduced. More reduction of the infarct area in the Skp2 group was observed than in the D1NLS group. Compared with the Cont group, the infarct area was at a smaller value in the Skp2 group.

Based on the above results, it was confirmed that the introduction of D1NLS + CDK4 + Skp2 genes ameliorated the deterioration of cardiac functions following the onset of cardiac infarction and the exacerbation of hemodynamics to suppress lung congestion and left ventricular remodeling and to additionally give an effect on the reduction of the area of cardiac infraction.

According to the method of the invention, cell division of cardiomyocytes can be more efficiently induced than by the related-art methods, to induce the proliferation of the cell. The cardiomyocytes prepared in such manner can be utilized as a cell for screening for various pharmaceutical drugs and for graft therapy. Additionally, the application of the method to gene therapy may expectedly lead to the application of regenerative medical therapy of cardiac diseases with underlining etiologies such as cardiomyocytes deficiency.

## Claims

**1.** A pharmaceutical composition comprising:

(a) a cyclin, said cyclin being selected from cyclin D1, cyclin D2, and cyclin D3,
(b) a cyclin-dependent kinase, said cyclin-dependent kinase being selected from CDK4 and CDK6, and
(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family protein;

wherein at least one of the gene encoding said cyclin and the gene encoding said cyclin-dependent kinase is tagged with a nucleotide sequence encoding a nuclear localization signal.

**2.** A method for proliferating cardiomyocytes comprising a step of introducing

(a) a cyclin, said cyclin being selected from cyclin D1, cyclin D2, and cyclin D3,
(b) a cyclin-dependent kinase, said cyclin-dependent kinase being selected from CDK4 and CDK6, and
(c) one or a plurality selected froth the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family protein

into cardiomyocytes in vitro; and
a step of subsequently culturing said cells;
wherein at least one of the gene encoding said cyclin and the gene encoding said cyclin-dependent kinase is tagged with a nucleotide sequence encoding a nuclear localization signal.

**3.** Use of

(a) a cyclin, said cyclin being selected from cyclin D1, cyclin D2, and cyclin D3,
(b) a cyclin-dependent kinase, said cyclin-dependent kinase being selected from CDK4 and CDK6, and

(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family protein
for the manufacture of a pharmaceutical composition for treating a cardiac disorder,
wherein at least one of the gene encoding said cyclin and the gene encoding said cyclin-dependent kinase is tagged with a nucleotide sequence encoding a nuclear localization signal.

**4.** The pharmaceutical composition of claim 1, the method of claim 2, or the use of claim 3, wherein the Cip/Kip family protein is $p27^{Kip1}$.

**5.** The method of claim 2, comprising introducing the genes into cardiomyocytes, using a viral vector or liposome.

**6.** A vector comprising

(a) a cyclin gene, the cyclin being selected from cyclin D1, cyclin D2, and cyclin D3,
(b) a cyclin-dependent kinase gene, the cyclin-dependent kinase being selected from CDK4 and CDK6, and
(c) one or a plurality selected from the group consisting of

(i) a gene encoding a factor that inhibits the production, function or action of Cip/Kip family protein, wherein said factor is a factor with an action to promote the degradation of the Cip/Kip family protein and is a component of ubiquitin ligase; and
(ii) a nucleic acid that inhibits the production of Cip/Kip family protein, wherein said nucleic acid is siRNA specific to a gene encoding the Cip/Kip family-protein,

wherein at least one of the gene encoding said cyclin and the gene encoding said cyclin-dependent kinase is tagged with a nucleotide sequence encoding a nuclear localization signal.

**7.** The pharmaceutical composition of claim 1 or 4, the method of any one of claims 2, 4 or 5, the use of claim 3 or 4, or the vector of claim 6, wherein the component of ubiquitin ligase is an F-box factor capable of binding to the Cip/Kip family protein.

**8.** The pharmaceutical composition, method, use or vector of claim 7, wherein the F-box factor capable of binding to the Cip/Kip family protein is Skp2.

**9.** The pharmaceutical composition of claim 1 or 4, the method of any one of claims 2, 4 or 5, the use of claim 3 or 4, or the vector of any one of claims 6 to 8, wherein the nucleic acid that inhibits the production of Cip/Kip family protein is siRNA that is specific to $p27^{Kip1}$ gene.

**10.** A pharmaceutical composition comprising the vector of any one of claims 6 to 9.

**11.** Use of the vector of any one of claims 6 to 9 for the manufacture of a pharmaceutical composition for treating a cardiac disorder.

**12.** The use of any one of claims 3, 4, 7 to 9 or 11, wherein the cardiac disorder is myocardial infarction, ischemic heart disease, congestive heart failure, hypertrophic cardiomyopathy, dilated cardiomyopathy, myocarditis, or chronic heart failure.

**Patentansprüche**

**1.** Arzneimittel, das umfasst:

(a) ein Cyclin, wobei das Cyclin ausgewählt ist aus Cyclin D1, Cyclin D2 und Cyclin D3,
(b) eine Cyclin-abhängige Kinase, wobei die Cyclin-abhängige Kinase ausgewählt ist aus CDK4 und CDK6, und

(c) ein Element oder eine Vielzahl von Elementen ausgewählt aus der Gruppe bestehend aus

(i) einem Gen, das einen Faktor kodiert, der die Produktion, Funktion oder Aktivität eines Proteins der Cip/Kip-Familie inhibiert, wobei der Faktor ein Faktor ist, der Aktivität zur Förderung des Abbaus des Proteins der Cip/Kip-Familie aufweist, und eine Komponente einer Ubiquitin-Ligase ist; und
(ii) einer Nukleinsäure, die die Produktion des Proteins der Cip/Kip-Familie inhibiert, wobei die Nukleinsäure eine siRNA ist, die spezifisch für ein Gen ist, das das Protein der Cip/Kip-Familie kodiert;

wobei mindestens eines der Gene, das das Cyclin kodiert, und das Gen, das die Cyclin-abhängige Kinase kodiert, mit einer Nukleotidsequenz markiert ist, die ein Kernlokalisierungssignal kodiert.

**2.** Verfahren zur Proliferation von Kardiomyozyten, umfassend einen Schritt zur Einführung von

(a) einem Cyclin, wobei das Cyclin ausgewählt ist aus Cyclin D1, Cyclin D2 und Cyclin D3,
(b) einer Cyclin-abhängigen Kinase, wobei die Cyclin-abhängige Kinase ausgewählt ist aus CDK4 und CDK6, und
(c) einem Element oder einer Vielzahl von Elementen ausgewählt aus der Gruppe bestehend aus

(i) einem Gen, das einen Faktor kodiert, der die Produktion, Funktion oder Aktivität des Proteins der Cip/Kip-Familie inhibiert, wobei der Faktor ein Faktor ist, der Aktivität zur Förderung des Abbaus des Proteins der Cip/Kip-Familie aufweist, und eine Komponente einer Ubiquitin-Ligase ist; und
(ii) einer Nukleinsäure, die die Produktion eines Proteins der Cip/Kip-Familie inhibiert, wobei die Nukleinsäure eine siRNA ist, die spezifisch für ein Gen ist, das das Protein der Cip/Kip-Familie kodiert;

in Kardiomyozyten in vitro; und
einen Schritt einer anschließenden Züchtung der Zellen;
wobei mindestens eines der Gene, das das Cyclin kodiert, und das Gen, das die Cyclin-abhängige Kinase kodiert, mit einer Nukleotidsequenz markiert ist, die ein Kernlokalisierungssignal kodiert.

**3.** Verwendung von

(a) einem Cyclin, wobei das Cyclin ausgewählt ist aus Cyclin D1, Cyclin D2 und Cyclin D3,
(b) einer Cyclin-abhängigen Kinase, wobei die Cyclin-abhängige Kinase ausgewählt ist aus CDK4 und CDK6, und
(c) einem Element oder einer Vielzahl von Elementen ausgewählt aus der Gruppe bestehend aus

(i) einem Gen, das einen Faktor kodiert, der die Produktion, Funktion oder Aktivität eines Proteins der Cip/Kip-Familie inhibiert, wobei der Faktor ein Faktor ist, der Aktivität zur Förderung des Abbaus des Proteins der Cip/Kip-Familie aufweist, und eine Komponente einer Ubiquitin-Ligase ist; und
(ii) einer Nukleinsäure, die die Produktion des Proteins der Cip/Kip-Familie inhibiert, wobei die Nukleinsäure eine siRNA ist, die spezifisch für ein Gen ist, das das Protein der Cip/Kip-Familie kodiert,

für die Herstellung eines Arzneimittels zur Behandlung einer Funktionsstörung des Herzens,
wobei mindestens eines der Gene, das das Cyclin kodiert, und das Gen, das die Cyclin-abhängige Kinase kodiert, mit einer Nukleotidsequenz markiert ist, die ein Kernlokalisierungssignal kodiert.

**4.** Arzneimittel nach Anspruch 1, Verfahren nach Anspruch 2 oder Verwendung nach Anspruch 3, wobei das Protein der Cip/Kip-Familie $p27^{Kip1}$ ist.

**5.** Verfahren nach Anspruch 2, umfassend die Einführung der Gene in Kardiomyozyten unter Verwendung eines viralen Vektors oder eines Liposoms.

**6.** Vektor, der umfasst

(a) ein Cyclingen, wobei das Cyclin ausgewählt ist aus Cyclin D1, Cyclin D2 und Cyclin D3,
(b) das Gen einer Cyclin-abhängigen Kinase, wobei die Cyclin-abhängige Kinase ausgewählt ist aus CDK4 und CDK6, und
(c) ein Element oder eine Vielzahl von Elementen ausgewählt aus der Gruppe bestehend aus

(i) einem Gen, das einen Faktor kodiert, der die Produktion, Funktion oder Aktivität eines Proteins der Cip/Kip-Familie inhibiert, wobei der Faktor ein Faktor ist, der Aktivität zur Förderung des Abbaus des Proteins der Cip/Kip-Familie aufweist, und eine Komponente einer Ubiquitin-Ligase ist; und
(ii) einer Nukleinsäure, die die Produktion des Proteins der Cip/Kip-Familie inhibiert, wobei die Nukleinsäure eine siRNA ist, die spezifisch für ein Gen ist, das das Protein der Cip/Kip-Familie kodiert;

wobei mindestens eines der Gene, das das Cyclin kodiert, und das Gen, das die Cyclin-abhängige Kinase kodiert, mit einer Nukleotidsequenz markiert ist, die ein Kernlokalisierungssignal kodiert.

**7.** Arzneimittel nach Anspruch 1 oder 4, Verfahren nach einem der Ansprüche 2, 4 oder 5, Verwendung nach Anspruch 3 oder 4 oder Vektor nach Anspruch 6, wobei die Komponente der Ubiquitin-Ligase ein F-Box-Faktor ist, der fähig ist, an das Protein der Cip/Kip-Familie zu binden.

**8.** Arzneimittel, Verfahren, Verwendung oder Vektor nach Anspruch 7, wobei der F-Box-Faktor, der fähig ist, an das Protein der Cip/Kip-Familie zu binden, Skp2 ist.

**9.** Arzneimittel nach Anspruch 1 oder 4, Verfahren nach einem der Ansprüche 2, 4 oder 5, Verwendung nach Anspruch 3 oder 4 oder Vektor nach einem der Ansprüche 6 bis 8, wobei die Nukleinsäure, die die Produktion des Proteins der Cip/Kip-Familie inhibiert, eine siRNA ist, die spezifisch für das $p27^{Kip1}$-Gen ist.

**10.** Arzneimittel, das den Vektor nach einem der Ansprüche 6 bis 9 umfasst.

**11.** Verwendung des Vektors nach einem der Ansprüche 6 bis 9 für die Herstellung eines Arzneimittels zur Behandlung einer Funktionsstörung des Herzens.

**12.** Verwendung nach einem der Ansprüche 3, 4, 7 bis 9 oder 11, wobei die Funktionsstörung des Herzens ein Myokardinfarkt, ischämische Herzkrankheit, dekompensierte Herzinsuffizienz, hypertrophische Kardiomyopathie, dilatative Kardiomyopathie, Myokarditis oder chronische Herzinsuffizienz ist.

## Revendications

**1.** Composition pharmaceutique comprenant :

(a) une cycline, ladite cycline étant choisie parmi la cycline D1, la cycline D2 et la cycline D3,
(b) une kinase cycline-dépendante, ladite kinase cycline-dépendante étant choisie parmi la CDK4 et la CDK6, et
(c) l'un ou une pluralité choisi dans le groupe constitué par

(i) un gène codant pour un facteur qui inhibe la production, la fonction ou l'action d'une protéine de la famille Cip/Kip, où ledit facteur est un facteur avec une action qui favorise la dégradation d'une protéine de la famille Cip/Kip et est un composant de l'ubiquitine-ligase ; et
(ii) un acide nucléique qui inhibe la production d'une protéine de la famille Cip/Kip où ledit acide nucléique est un ARNsi spécifique d'un gène codant pour une protéine de la famille Cip/Kip ;

dans laquelle au moins un parmi le gène codant pour ladite cycline et le gène codant pour ladite kinase cycline-dépendante est marqué par une séquence nucléotidique codant pour un signal de localisation nucléaire.

**2.** Procédé permettant la prolifération de cardiomyocytes comprenant une étape d'introduction

(a) d'une cycline, ladite cycline étant choisie parmi la cycline D1, la cycline D2 et la cycline D3,
(b) d'une kinase cycline-dépendante, ladite kinase cycline-dépendante étant choisie parmi la CDK4 et la CDK6, et
(c) d'un ou d'une pluralité choisi dans le groupe constitué par

(i) un gène codant pour un facteur qui inhibe la production, la fonction ou l'action d'une protéine de la famille Cip/Kip, où ledit facteur est un facteur avec une action qui favorise la dégradation d'une protéine de la famille Cip/Kip et est un composant de l'ubiquitine-ligase ; et
(ii) un acide nucléique qui inhibe la production d'une protéine de la famille Cip/Kip où ledit acide nucléique est un ARNsi spécifique d'un gène codant pour une protéine de la famille Cip/Kip ;

dans des cardiomyocytes *in vitro* ; et
une étape consistant à cultiver ensuite lesdites cellules ;
dans lequel au moins un parmi le gène codant pour ladite cycline et le gène codant pour ladite kinase cycline-dépendante est marqué par une séquence nucléotidique codant pour un signal de localisation nucléaire.

**3.** Utilisation

(a) d'une cycline, ladite cycline étant choisie parmi la cycline D1, la cycline D2 et la cycline D3,
(b) d'une kinase cycline-dépendante, ladite kinase cycline-dépendante étant choisie parmi la CDK4 et la CDK6, et
(c) d'un ou d'une pluralité choisi dans le groupe constitué par

(i) un gène codant pour un facteur qui inhibe la production, la fonction ou l'action d'une protéine de la famille Cip/Kip, où ledit facteur est un facteur avec une action qui favorise la dégradation d'une protéine de la famille Cip/Kip et est un composant de l'ubiquitine-ligase ; et
(ii) un acide nucléique qui inhibe la production d'une protéine de la famille Cip/Kip où ledit acide nucléique est un ARNsi spécifique d'un gène codant pour une protéine de la famille Cip/Kip ;

pour la fabrication d'une composition pharmaceutique destinée au traitement d'un trouble cardiaque,
dans laquelle au moins un parmi le gène codant pour ladite cycline et le gène codant pour ladite kinase cycline-dépendante est marqué par une séquence nucléotidique codant pour un signal de localisation nucléaire.

**4.** Composition pharmaceutique selon la revendication 1, procédé selon la revendication 2 ou utilisation selon la revendication 3, où la protéine de la famille Cip/Kip est la $p27^{Kip1}$.

**5.** Procédé selon la revendication 2, comprenant l'introduction des gènes dans des cardiomyocytes en utilisant un vecteur viral ou un liposome.

**6.** Vecteur comprenant

(a) un gène de cycline, ladite cycline étant choisie parmi la cycline D1, la cycline D2 et la cycline D3,
(b) un gène de kinase cycline-dépendante, ladite kinase cycline-dépendante étant choisie parmi la CDK4 et la CDK6, et
(c) l'un ou une pluralité choisi dans le groupe constitué par

(i) un gène codant pour un facteur qui inhibe la production, la fonction ou l'action d'une protéine de la famille Cip/Kip, où ledit facteur est un facteur avec une action qui favorise la dégradation d'une protéine de la famille Cip/Kip et est un composant de l'ubiquitine-ligase ; et
(ii) un acide nucléique qui inhibe la production d'une protéine de la famille Cip/Kip où ledit acide nucléique est un ARNsi spécifique d'un gène codant pour une protéine de la famille Cip/Kip ;

dans lequel au moins un parmi le gène codant pour ladite cycline et le gène codant pour ladite kinase cycline-dépendante est marqué par une séquence nucléotidique codant pour un signal de localisation nucléaire.

**7.** Composition pharmaceutique selon la revendication 1 ou 4, procédé selon l'une quelconque des revendications 2, 4 ou 5, utilisation selon la revendication 3 ou 4 ou vecteur selon la revendication 6, où le composant de l'ubiquitine-ligase est un facteur F-box capable de se lier à une protéine de la famille Cip/Kip.

**8.** Composition pharmaceutique, procédé, utilisation ou vecteur selon la revendication 7, où le facteur F-box capable de se lier à une protéine de la famille Cip/Kip est Skp2.

**9.** Composition pharmaceutique selon la revendication 1 ou 4, procédé selon l'une quelconque des revendications 2, 4 ou 5, utilisation selon la revendication 3 ou 4 ou vecteur selon l'une quelconque des revendications 6 à 8, où l'acide nucléique qui inhibe la production d'une protéine de la famille Cip/Kip est un ARNsi qui est spécifique du gène $p27^{Kip1}$.

**10.** Composition pharmaceutique comprenant le vecteur selon l'une quelconque des revendications 6 à 9.

**11.** Utilisation du vecteur selon l'une quelconque des revendications 6 à 9 pour la fabrication d'une composition pharmaceutique destinée au traitement d'un trouble cardiaque.

**12.** Utilisation selon l'une quelconque des revendications 3, 4, 7 à 9 ou 11, dans laquelle le trouble cardiaque est un infarctus du myocarde, une cardiopathie ischémique, une insuffisance cardiaque congestive, une cardiomyopathie hypertrophique, une cardiomyopathie dilatée, une myocardite ou une insuffisance cardiaque chronique.

Fig. 1

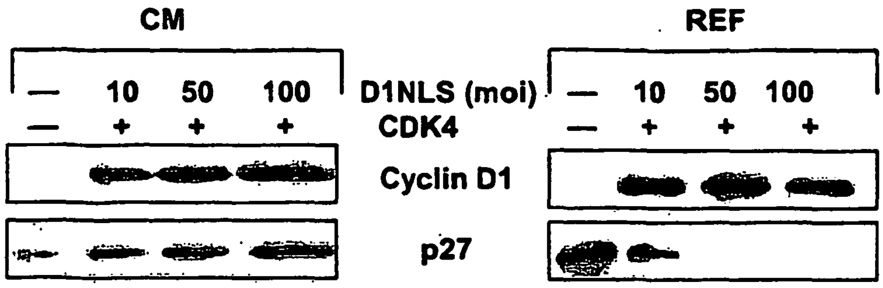


Fig. 2

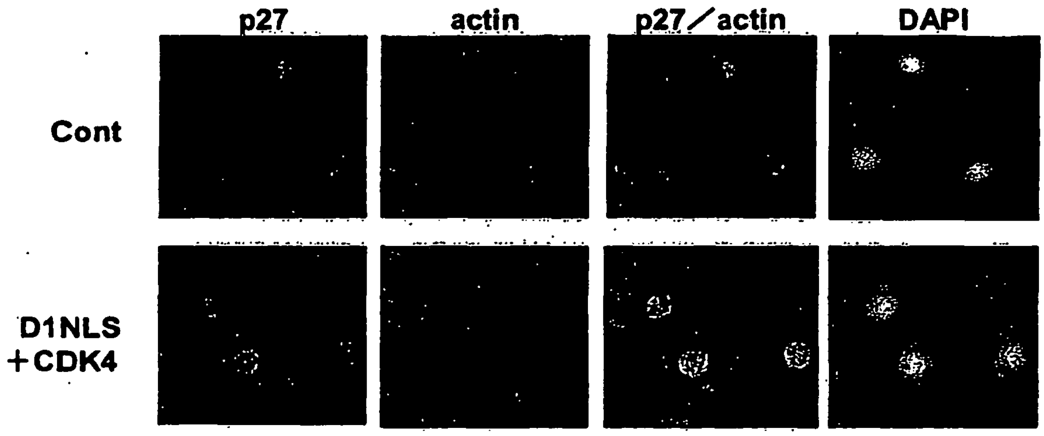


Fig. 3

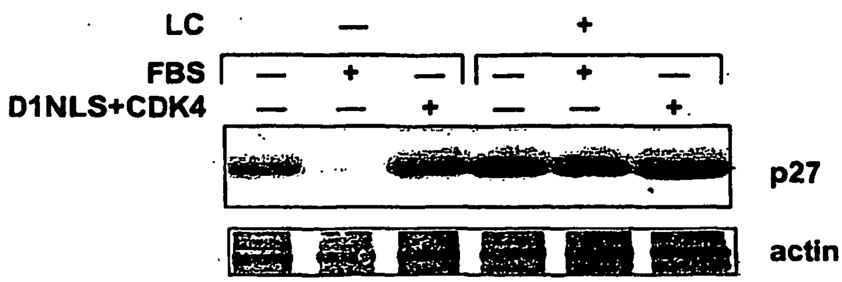

Fig. 4

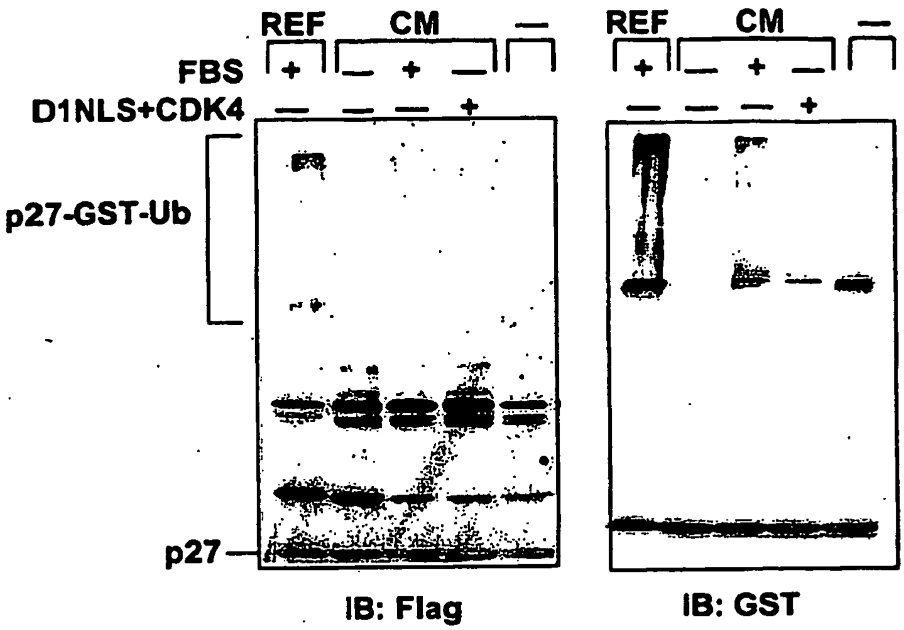


Fig. 5

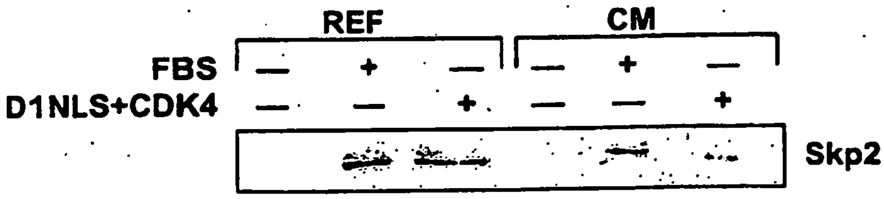


Fig. 6

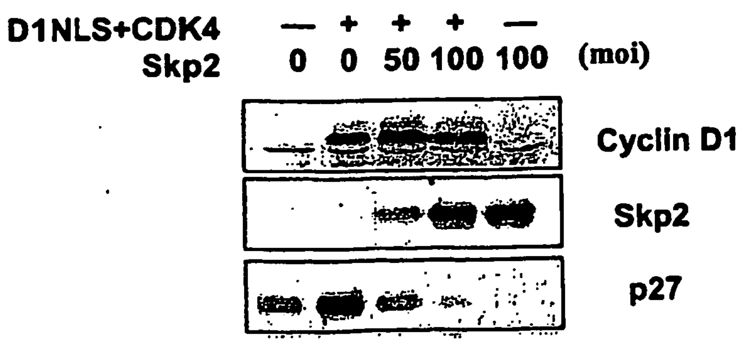

Fig. 7

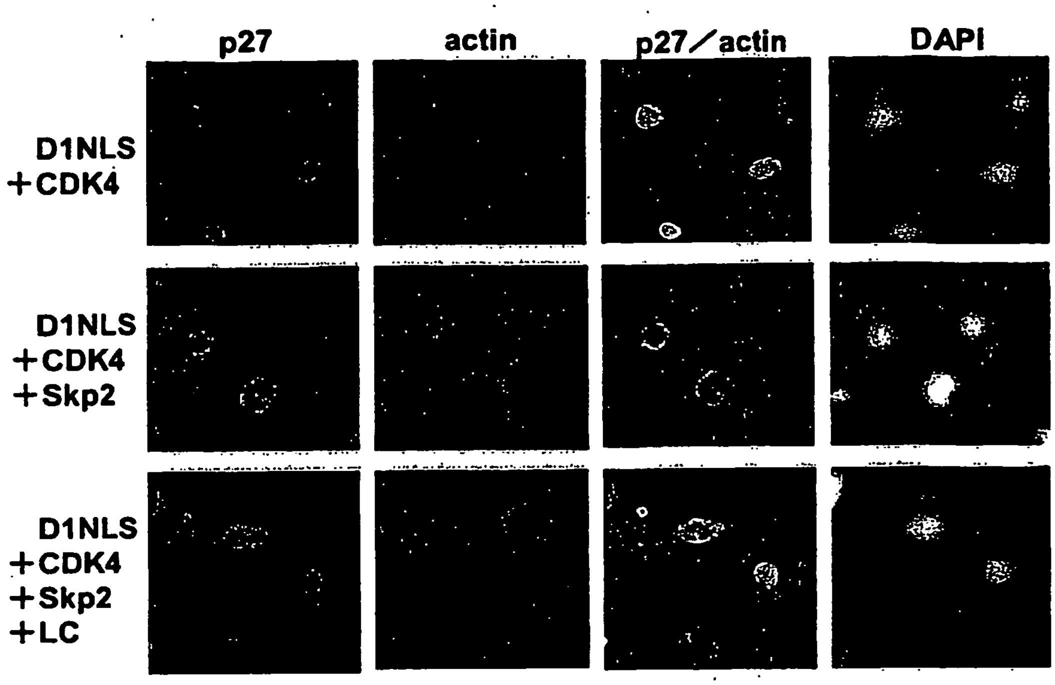
p27
actin
p27/actin
DAPI
D1NLS
+CDK4
D1NLS
+CDK4
+Skp2
D1NLS
+CDK4
+Skp2
+LC

Fig. 8

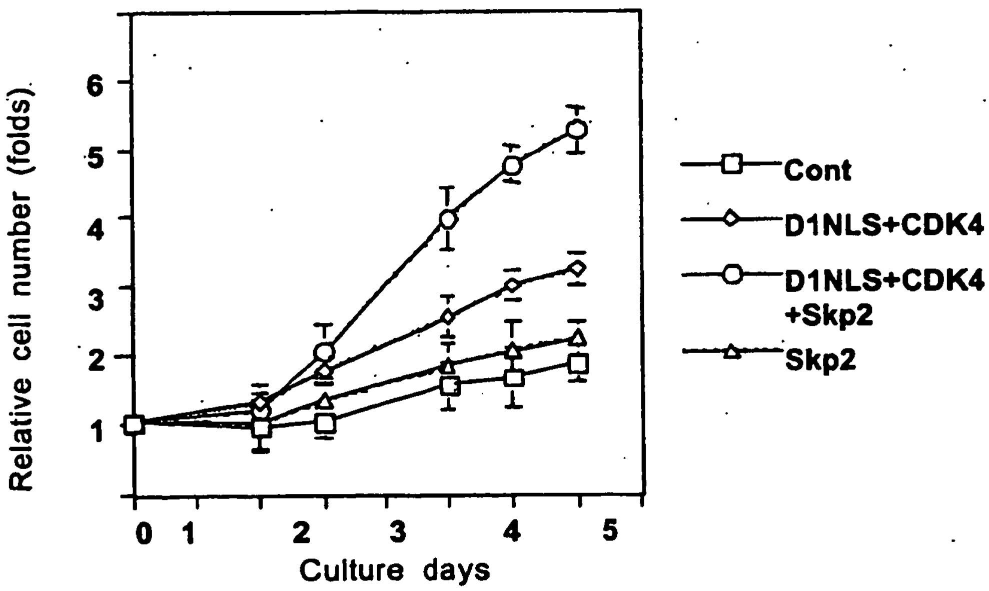
Relative cell number (folds)
1
2
3
4
5
6
0
1
2
3
4
5
Culture days
Cont
D1NLS+CDK4
D1NLS+CDK4
+Skp2
Skp2

Fig. 9

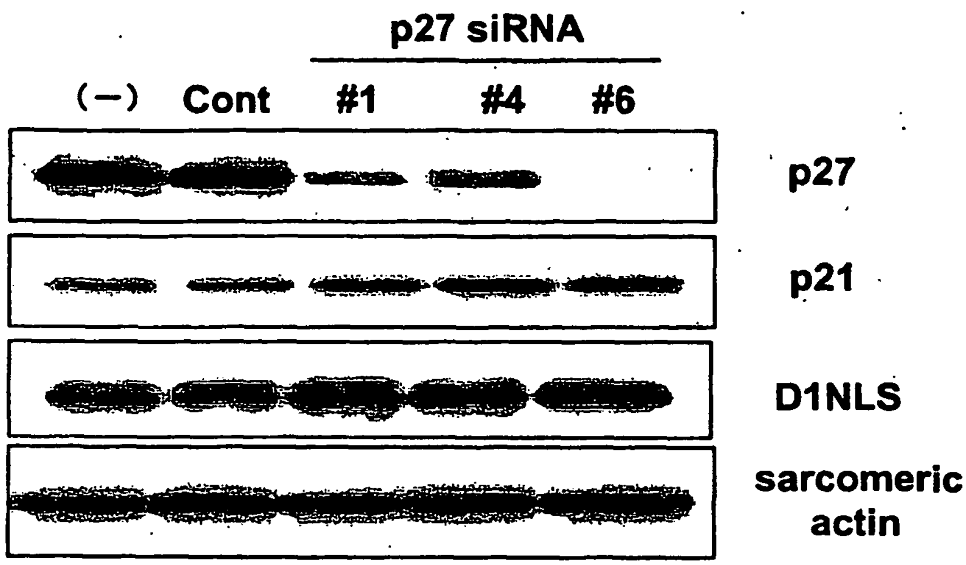

Fig. 10

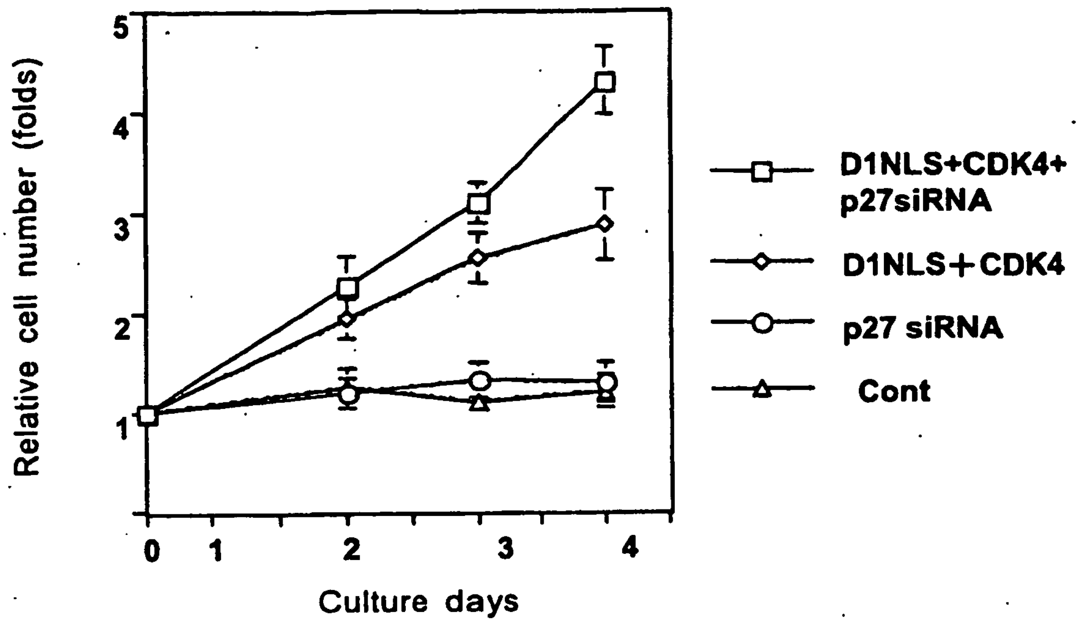
Relative cell number (folds)
5
4
3
2
1
0
1
2
3
4
Culture days
D1NLS+CDK4+ p27siRNA
D1NLS+CDK4
p27 siRNA
Cont

Fig. 11

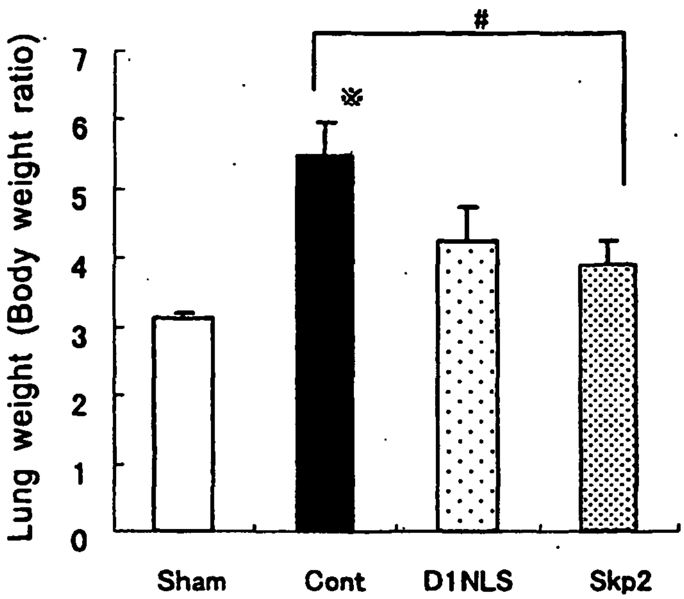
#
※
Lung weight (Body weight ratio)
7
6
5
4
3
2
1
0
Sham
Cont
D1NLS
Skp2

Fig. 12

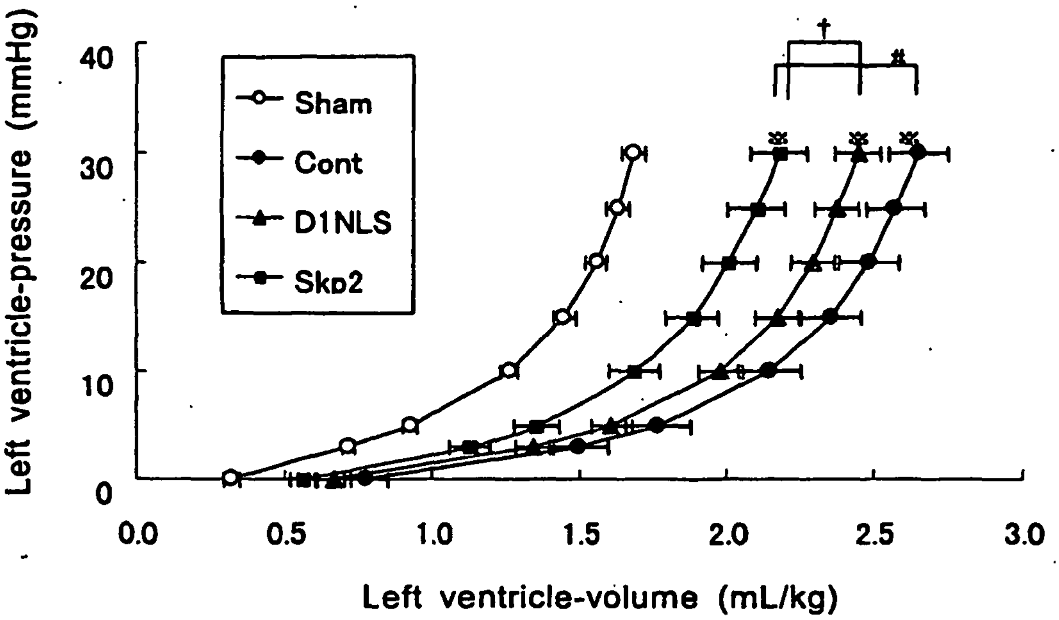
Left ventricle-pressure (mmHg)
40
30
20
10
0
0.0
0.5
1.0
1.5
2.0
2.5
3.0
Left ventricle-volume (mL/kg)
Sham
Cont
D1NLS
Skp2
†
#
※

Fig. 13

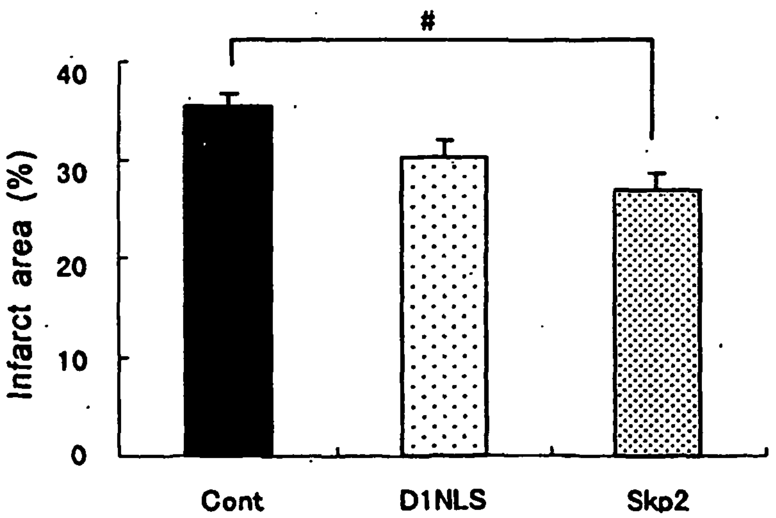
Infarct area (%)
40
30
20
10
0
#
Cont
D1NLS
Skp2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02095026 A
- US 6090622 A
- US 0111011 A
- US 02051980 A
- US P5736396 A, Bruder
- WO 01048151 A
- WO 03035382 A
- US P6015671 A, Field
- WO 0306950 A, Xu
- JP 2001224380 A, Nakayama
- EP 926236 A, Buergin
- US P6265562 A, Buergin
- WO 0015821 A
- US 6015671 A
- JP P2003391708 B
- JP P2004246533 B
- JP 2004017274 W


### Non-patent literature cited in the description


- **Li ; Brooks.** *European Heart Journal,* 1999, vol. 20, 406-420
- **Soonpaa et al.** *Science,* 1994, vol. 264, 98
- **Murry et al.** *Cold Spring Harb. Symp. Quant. Biol.,* 2002, vol. 67, 519
- **Menasche.** *Ann. Thorac. Surg.,* 2003, vol. 75, 20
- **Nir et al.** *Cardiovasc. Res.,* 2003, vol. 58, 313
- **Tamamori-Adachi et al.** *Circ. Res.,* 2003, vol. 92, e12
- **Kishore et al.** *Circ. Res.,* 2002, vol. 90, 1044
- **Kirshenbaum et al.** *J. Biol. Chem.,* 1995, vol. 270, 7791
- **Kirshenbaum et al.** *Dev. Biol.,* 1996, vol. 179, 402
- **Soonpaa et al.** *Clin. Invest.,* 1997, vol. 99, 2644
- **Toyoda et al.** *Dev. Cell,* 2003, vol. 5, 85
- **Sherr ; Roberts.** *Genes Dev.,* 1995, vol. 9, 1149
- **Hannon ; Beach.** *Nature,* 1993, vol. 371, 257
- **Serrano et al.** *Nature,* 1993, vol. 366, 704
- **Hirai et al.** *Mol. Cell. Biol.,* 1995, vol. 15, 2672
- **Harper et al.** *Cell,* vol. 75, 805
- **Polyyak et al.** *Cell,* 1994, vol. 78, 59
- **Toyoshima ; Hunter.** *Cell,* 1994, vol. 78, 67
- **Matsuoka et al.** *Genes Dev.,* 1995, vol. 9, 650
- **Flink et al.** *J. Mol. Cell. Cardiol.,* 1998, vol. 30, 563
- **von Harsdorf et al.** *Circ. Res.,* 1999, vol. 85, 128
- **Poolman et al.** *Circ. Res.,* 1999, vol. 85, 117
- **Pagano et al.** *Science,* 1995, vol. 269, 682
- **Maki ; Howley.** *Mol. Cell Biol.,* 1997, vol. 17, 355
- **Urano et al.** *J. Biol. Chem.,* 1999, vol. 274, 12197
- **Coux et al.** *Annu. Rev. Biochem.,* 1996, vol. 65, 801
- **Hochstrasser.** *Annu. Rev. Genet.,* 1996, vol. 30, 405
- **Pagano.** *FASEV J.,* 1997, vol. 11, 1067
- **Hershko et al.** *Annu. Rev. Biochem.,* 1998, vol. 67, 425
- **Patton et al.** *Trends Genet.,* 1998, vol. 14, 236
- **Jackson ; Eldridge.** *Mol. Cell,* 2002, vol. 9, 923
- **Bai et al.** *Cell,* 1996, vol. 86, 263
- **Slowrya et al.** *Cell,* 1997, vol. 91, 209
- **Kobe et al.** *Curr. Opin. Struct. Biol.,* 2001, 11725
- **Carrano et al.** *Nature Cell Biol.,* 1999, vol. 1, 193
- **Tsverkov et al.** *Curr. Biol.,* 1999, vol. 9, 661
- **Bornstein et al.** *J. Biol . Chem.,* 2003, vol. 278, 26752
- **Kamura et al.** *Proc. Natl . Acad. Sci. USA,* 2003, vol. 100, 10231
- **Zhang et al.** *Cell,* 1995, vol. 82, 915
- **Marti et al.** *Nat. Cell Biol.,* 1999, vol. 1, 14
- **Nakayama et al.** *EMBO J.,* 2000, vol. 19, 2069
- **Kiernan et al.** *Mol. Cell. Biol.,* 2001, vol. 21, 7956
- **Kim et al.** *Cell,* 2003, vol. 11, 1177
- **von der Lehr et al.** *Mol. Cell,* 2003, vol. 11, 1189
- Molecular Cloning: A Laboratory Manual. Cold Spring Harbor Laboratory Press, 2001

- Current Protocols in Molecular biology. John Wiley & Sons, 1987
- Methods in Enzymology in series. Academic Press
- PCR Protocols: Methods in Molecular Biology. Humana Press, 2003
- Animal Cell Culture: A Practical Approach. Oxford University Press, 2000
- Antibodies: A Laboratory Manual. Cold Spring Harbor Laboratory Press, 1987
- **Chien et al.** *J. Clin. Invest.,* 1985, vol. 75, 1770
- **Meidell et al.** *Am. J. Physiol.,* 1986, vol. 251, H1076
- **Tamamori et al.** *Am. J. Physiol.,* 1998, vol. 275, H2036
- Mouse Embryo: A laboratory manual. Cold Spring Harbor Laboratory Press, 1994
- Embryonic Stem Cells. Humana Press, 2002
- **Matsui et al.** *Cell,* 1992, vol. 70, 841
- **Shamblott et al.** *Proc. Nat1. Acad. Sci. USA,* 1998, vol. 95, 13726
- **Jiang et al.** *Nature,* 2002, vol. 418, 41
- **Abuljadayel.** *Curr. Med. Res. Opinion,* 2003, vol. 19, 355
- **Li et al.** *Nature Med.*
- **Pittenger et al.** *Science,* 1999, vol. 284, 143
- **Makino et al.** *J. Clin. Invest.,* 1999, vol. 103, 697
- Embryonic Stem Cells. Human Press, 2002
- **Klug et al.** *J. Clin. Invest.,* 1996, vol. 98, 216
- **Wobus et al.** *J. Mol. Cell. Cardiol.,* 1997, vol. 29, 1525
- **Kehat et al.** *J. Clin. Invest.,* 2001, vol. 108, 407
- **Xu et al.** *Circ. Res.,* 2002, vol. 91, 501
- **Takahashi et al.** *Circulation,* 2003, vol. 107, 1912
- **Davy et al.** *Cell,* 1985, vol. 40, 667
- **Kalderon et al.** *Nature,* 1984, vol. 311, 33
- **Robbin et al.** *Cell,* 1991, vol. 64, 615
- **Koff et al.** *Cell,* 1991, vol. 66, 1217
- **Polyak et al.** *Cell,* 1994, vol. 79, 59
- **Nourse et al.** *Nature,* 1994, vol. 372, 570
- **Mainprize et al.** *J. Neurooncol.,* 2001, vol. 51, 205
- **Conqueret.** *Trends. Cell Biol.,* 2003, vol. 13, 65
- **Pagono et al.** *Science,* 1995, vol. 269, 682
- **Carrano et al.** *Nature Cell Biol .,* 1999, vol. 1, 193
- **Tsvetkov et al.** *Curr. Biol.,* 1999, vol. 9, 661
- **Bornstein et al.** *J. Biol. Chem.,* 2003, vol. 278, 26752
- **Kamura et al.** *Proc. Natl. Acad. Sci. USA,* 2003, vol. 100, 10231
- **Mueller et al.** *EMBO J.,* 2000, vol. 19, 2168
- **Smitherman et al.** *Mol. Cell. Biol.,* 2000, vol. 20, 5631
- **Tomoda et al.** *J. Biol. Chem.,* 2002, vol. 277, 2302
- **Ishida et al.** *J Biol. Chem.,* 2002, vol. 277, 14355
- **Connor et al.** *Mol. Biol. Cell,* 2003, vol. 14, 201
- **Boehm et al.** *EMBO J.,* 2002, vol. 21, 3390
- **Niwa et al.** *Gene,* 1991, vol. 108, 193
- **Lee et al.** *J. Biol. Chem.,* 1992, vol. 267, 15876
- **Cuo et al.** *Development,* 1999, vol. 126, 4223
- **Hammond et al.** *Nat. Rev. Genet.,* 2001, vol. 2, 110
- **Elbashir et al.** *Nature,* 2002, 411-494
- RNA Interference (RNAi) : The Nuts & Bolts of siRNA Technology. DNA Press, 2004
- RNA Interference, Editing, and Modification; Methods and Protocols. Humana Press, 2004
- **Chalk et al.** *Biochem. Biophys. Res. Commun.,* 2004, vol. 319, 264
- **Ti-Tei et al.** *Nucl. Acids Res.,* 2004, vol. 32, 936
- **Reynolds et al.** *Nat. Biotechnol.,* 2004, vol. 22, 326
- **Miyake et al.** *Proc. Natl. Acad. Sci. USA,* 1996, vol. 93, 1320
- **Mizuguchi et al.** *Hum. Gene Ther.,* 1998, vol. 9, 2577
- Gene Therapy Protocols: Methods in Molecular Medicine. Humana Press, 1997
- Gene Transfer in Cardiovascular System: Experimental Approaches & Therapeutic Implications. Kluwer Academic Publishers, 1997
- Adenoviral Vectors for Gene Therapy. Academic Press, 2002
- **Banerjee, J.** *Biomater. Appl.,* 2001, vol. 16, 3
- **Maurer et al.** *Expert Opin. Biol. Ther.,* 2001, vol. 1, 923
- **Yonemitsu et al.** *Int. J. Oncol.,* 1998, vol. 12, 1277
- **Kaneda et al.** *Mol. Med. Today,* 1999, vol. 5, 298
- Vascular Surgery. W.B.Saunders, 2000
- Textbook of Interventional Cardiology. W.B.Saunders, 2002
- *J. Clin. Invest.,* 1996, vol. 98, 216
- **Shimizu et al.** *Circ. Res.,* 2002, vol. 90, e40
- **Dowell et al.** *Cardiovasc. Res.,* 2003, vol. 58, 336
- **Reffelmann et al.** *Heart Fail. Rev.,* 2003, vol. 8, 201
- **Maltsev et al.** *Mech. Dev.,* 1993, vol. 44, 41
- *Circ. Res.,* 1994, vol. 75, 233
- **Dr. Sander van den Heuvel.** *Massachusetts General Hospital Cancer Center*

- **van den Heuvel et al.** *Science,* 1993, vol. 262, 2050
- **Matsushime et al.** *Cell,* 1991, vol. 65, 701
- **Hara et al.** *J. Biol. Chem.,* 2001, vol. 276, 48937
- **Ishida et al.** *J. Biol. Chem.,* 2002, vol. 277, 14355
- **Tsverkiv et al.** *Curr. Biol.,* 1999, vol. 9, 661
- **Dairaku et al.** *Circ. J.,* 2002, vol. 66, 411
- **O'brien et al.** *Lab. Anim. Sci.,* 1997, vol. 47, 486
- **Morimoto et al.** *J. Pharmacol. Sci.,* 2003, vol. 91 (1), 151
- **Pfeffer et al.** *Circ. Res.,* 1985, vol. 57, 84
- **Jain et al.** *Circulation,* 2002, vol. 103, 1920

SEQUENCE LISTING

<110> Daiichi Asubio Pharma Co., Ltd.
Mimi, Adachi
Keiichi, Nakayama
Shigetaka, Kitajima
Hiromitsu, Takagi

<120> Method for proliferating cardiomyocytes

<130> M 1833 EP

<150> JP P2003-391708
<151> 2003-11-21

<150> JP P2004-246533
<151> 2004-08-26

<150> PCT/JP2004/017274
<151> 2004-11-19

<160> 15

<170> PatentIn version 3.1

<210> 1
<211> 8 .
<212> PRT

<213> Simian virus 40

<400> 1

Pro Pro Lys Lys Lys Arg Lys Val
1               5

<210> 2
<211> 23
<212> DNA
<213> Artificial Sequence

<220>
<223> 5' primer of C-terminal cyclin D1

<400> 2
accctccatg gtagctgctg gga 23

<210> 3
<211> 33
<212> DNA
<213> Artificial Sequence

<220>
<223> 3' primer of C-terminal cyclin D1

<400> 3
tgatctcgag gtcgatgtcc acatctcgca cgt 33

<210> 4
<211> 8
<212> PRT
<213> Artificial Sequence

<220>
<223> Flag tag sequence

<400> 4

Asp Tyr Lys Asp Asp Asp Asp Lys
1               5

<210> 5
<211> 66
<212> DNA
<213> Artificial Sequence

<220>
<223> 5' primer of Skp2 cDNA

<400> 5

atactcgagg ccaccatgga ctacaaggac gacgatgaca agcataggaa gcaccttcag 60

gagatt 66

<210> 6
<211> 33
<212> DNA
<213> Artificial Sequence

<220>
<223> 3' primer of Skp2 cDNA

<400> 6
atactcgagt catagacaac tgggcttttg cag 33

<210> 7
<211> 18
<212> DNA
<213> Artificial Sequence

<220>
<223> Rat p27/Kip1 siRNA target sequence 1

<400> 7
ggcagaagat tcttcttc 18

<210> 8
<211> 19
<212> DNA
<213> Artificial Sequence

<220>
<223> Rat p27/Kip1 siRNA target sequence 2

<400> 8
agcgcaagtg gaatttcga 19

<210> 9
<211> 19
<212> DNA
<213> Artificial Sequence

<220>
<223> Rat p27/Kipl siRNA target sequence 3

<400> 9
gtgagagtgt ctaacggga 19

<210> 10
<211> 62
<212> DNA
<213> Artificial Sequence

<220>
<223> Oligo 1 for p27 siRNA-#1 expression

<400> 10

caccggtagg aggttcttct tcaacgtgtg ctgtccgttg aagaagaatc ttctgccttt 60

tt 62

<210> 11
<211> 62
<212> DNA
<213> Artificial Sequence

<220>
<223> Oligo 2 for p27 siRNA-#1 expression

<400> 11

gcataaaaag gcagaagatt cttcttcaac ggacagcaca cgttgaagaa gaacctccta 60

cc 62

<210> 12
<211> 62
<212> DNA
<213> Artificial Sequence

<220>
<223> Oligo 1 for p27 siRNA #4 expression

<400> 12

caccagtgta agtggagttt cgaacgtgtg ctgtccgttc gaaattccac ttgcgctttt 60

tt 62

<210> 13
<211> 62
<212> DNA
<213> Artificial Sequence

<220>
<223> Oligo 2 for p27 siRNA #4 expression

<400> 13

gcataaaaaa gcgcaagtgg aatttcgaac ggacagcaca cgttcgaaac tccacttaca 60

ct 62

<210> 14
<211> 62
<212> DNA
<213> Artificial Sequence

<220>
<223> Oligo 1 for p27 siRNA #6 expression

<400> 14

```
caccgtggga gtgtttaatg ggaacgtgtg ctgtccgttc ccgttagaca ctctcacttt      60

tt                                                                     62
```

<210> 15
<211> 62
<212> DNA
<213> Artificial Sequence

<220>
<223> Oligo 2 for p27 siRNA #6 expression

<400> 15

```
gcataaaaag tgagagtgtc taacgggaac ggacagcaca cgttcccatt aaacactccc      60

ac                                                                     62
```